(19) Europäisches Patentamt
European Patent Office
Office européen des brevets

(11) Veröffentlichungsnummer: **0 171 362 A1**

# (12) EUROPÄISCHE PATENTANMELDUNG

(21) Anmeldenummer: **85810360.9**

(22) Anmeldetag: **05.08.85**

(51) Int. Cl.[4]: **C 07 D 499/00**, A 61 K 31/43 // C07F9/65, C07D205/08

(30) Priorität: **09.08.84 CH 3819/84**

(43) Veröffentlichungstag der Anmeldung: **12.02.86 Patentblatt 86/7**

(84) Benannte Vertragsstaaten: **AT BE CH DE FR GB IT LI LU NL SE**

(71) Anmelder: **CIBA-GEIGY AG, Klybeckstrasse 141, CH-4002 Basel (CH)**

(72) Erfinder: **Lang, Marc, Dr., Rue des Ormes 6, F-68170 Rixheim (FR)**

(54) **Neue Aminoalkylpenem-Verbindungen.**

(57) Verbindungen der Formel

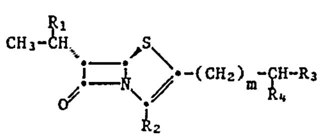

(I)

worin $R_1$ Hydroxy oder geschütztes Hydroxy ist, $R_2$ Carboxyl oder funktionell abgewandeltes Carboxyl bedeutet, $R_3$ Amino, durch Niederalkyl substituiertes Amino, Niederalkanoylamino, substituiertes Methylenamino oder geschütztes Amino ist, $R_4$ gegebenenfalls durch Amino, durch Niederalkyl substituiertes Amino, substituiertes Methylenamino, geschütztes Amino, Hydroxy oder geschütztes Hydroxy substituiertes Methyl oder Äthyl ist, und m eine ganze Zahl von 1 bis 3 ist, optische Isomere von Verbindungen der Formel I, Mischungen solcher optischen Isomere und Salze von solchen Verbindungen der Formel I, die eine salzbildende Gruppe aufweisen, besitzen antibiotische Eigenschaften. Die neuen Verbindungen können z. B. in Form von antibiotisch wirksamen Präparaten zur Behandlung von Infektionskrankheiten verwendet werden. Die neuen Verbindungen können in an sich bekannter Weise hergestellt werden.

EP 0 171 362 A1

CIBA-GEIGY AG
Basel (Schweiz)

4-15030/-

## Neue Aminoalkylpenem-Verbindungen

Die vorliegende Erfindung betrifft neue 2-Aminoniederalkyl-penem-Verbindungen, Verfahren zu ihrer Herstellung, pharmazeutische Präparate, die solche Verbindungen enthalten, und ihre Verwendung zur Herstellung von pharmazeutischen Präparaten oder als pharmakologisch wirksame Verbindungen.

Die Erfindung betrifft insbesondere 2-Aminoniederalkyl-2-penem-Verbindungen der Formel

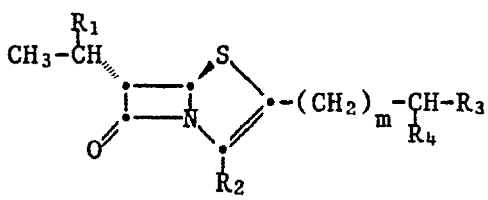

(I) ,

worin $R_1$ Hydroxy oder geschütztes Hydroxy ist, $R_2$ Carboxyl oder funktionell abgewandeltes Carboxyl bedeutet, $R_3$ Amino, durch Niederalkyl substituiertes Amino, Niederalkanoylamino, substituiertes Methylenamino oder geschütztes Amino ist, $R_4$ gegebenenfalls durch Amino, durch Niederalkyl substituiertes Amino, substituiertes Methylenamino, geschütztes Amino, Hydroxy oder geschütztes Hydroxy substituiertes Methyl oder Aethyl ist, und m eine ganze Zahl von 1 bis 3 ist, optische Isomere von Verbindungen der Formel I, Mischungen solcher optischen Isomere und Salze von solchen Verbindungen der Formel I, die eine salzbildende Gruppe aufweisen.

Die vor- und nachstehend verwendeten Definitionen haben im Rahmen der vorliegenden Beschreibung vorzugsweise die folgenden Bedeutungen:

Funktionell abgewandeltes Carboxy $R_2$ ist z.B. geschütztes Carboxyl $R_2'$ oder unter physiologischen Bedingungen spaltbares verestertes Carboxyl.

Eine unter physiologischen Bedingungen spaltbare veresterte Carboxylgruppe schützt die Verbindungen der Formel I vor Salzbildung im Magen-Darm-Trakt bei oraler Verabreichung, womit die vorzeitige Exkretion verhindert wird, und ist in erster Linie eine Acyloxymethoxycarbonylgruppe oder auch Acylaminomethoxycarbonylgruppe, worin Acyl z.B. den Rest einer organischen Carbonsäure, in erster Linie einer gegebenenfalls, z.B. durch Amino, substituierten Niederalkancarbonsäure oder Arencarbonsäure, z.B. Benzoesäure, bedeutet oder worin Acyloxymethyl den Rest eines Lactons bildet. Solche Gruppen sind z.B. Niederalkanoyloxymethoxycarbonyl, Aminoniederalkanoyloxymethoxycarbonyl, insbesondere α-Aminoniederalkanoyloxymethoxycarbonyl, Niederalkanoylaminomethoxycarbonyl, Benzaminomethoxycarbonyl, 4-Crotonolactonyl und 4-Butyrolacton-4-yl. Weitere unter physiologischen Bedingungen spaltbare veresterte Carboxylgruppen sind z.B. 5-Indanyloxycarbonyl, Phthalidyloxycarbonyl, 1-Niederalkoxycarbonyloxyniederalkoxycarbonyl, 1-Niederalkoxyniederalkoxycarbonyl oder 2-Oxo-1,3-dioxolen-4-ylmethoxycarbonyl, welches in 5-Stellung des Dioxolenringes gegebenenfalls durch Niederalkyl oder Phenyl substituiert ist.

Durch Niederalkyl substituiertes Amino ist z.B. Niederalkylamino oder Diniederalkylamino.

In substituiertem Methylenamino ist der Methylenrest vorzugsweise mono- oder disubstituiert. Substituiertes Methylenamino ist z.B. eine Gruppe der Formel

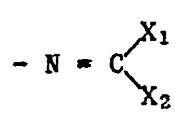

(IA) ,

worin $X_1$ Wasserstoff, gegebenenfalls substituiertes Amino, z.B. Amino, Niederalkylamino, Diniederalkylamino, Niederalkylenamino, Nitroamino, Hydrazino oder Anilino, veräthertes Hydroxy, z.B. Niederalkoxy oder Phenylniederalkoxy, veräthertes Mercapto, z.B. Niederalkylthio, gegebenenfalls substituiertes Niederalkyl, z.B. Niederalkyl, Aminoniederalkyl, N-Niederalkylaminoniederalkyl oder N,N-Diniederalkylaminoniederalkyl, Niederalkenyl, Phenyl oder über ein Ringkohlenstoffatom gebundenes monocyclisches Heteroaryl, wie entsprechendes 5- oder 6-gliedriges Heteroaryl mit 1 bis 2 Stickstoffatomen und/oder einem Sauerstoff-oder Schwefelatom, wie Pyridyl, z.B. 2- oder 4-Pyridyl, Thienyl, z.B. 2-Thienyl, oder Thiazolyl, z.B. 4-Thiazolyl, ist, und $X_2$ gegebenenfalls substituiertes Amino, z.B. Amino, Niederalkylamino, Diniederalkylamino, Niederalkylenamino, Hydrazino oder Anilino, veräthertes Hydroxy, z.B. Niederalkoxy oder Phenylniederalkoxy, oder veräthertes Mercapto, z.B. Niederalkylthio, darstellt.

In der vorliegenden Beschreibung bedeutet der im Zusammenhang mit Definitionen von Gruppen und Verbindungen verwendete Ausdruck "Nieder", dass die entsprechenden Gruppen bzw. Verbindungen, sofern nicht ausdrücklich anders definiert, bis zu 7, bevorzugt bis zu 4 Kohlenstoffatome enthalten.

Niederalkanoyloxymethoxycarbonyl ist z.B. Acetoxymethoxycarbonyl oder Pivaloyloxymethoxycarbonyl.

α-Aminoniederalkanoyloxymethoxycarbonyl ist z.B. Glycyloxymethoxycarbonyl, L-Valyloxymethoxycarbonyl oder L-Leucyloxymethoxycarbonyl.

Niederalkanoylaminomethoxycarbonyl ist z.B. Acetaminomethoxycarbonyl.

1-Niederalkoxycarbonyloxyniederalkoxycarbonyl ist z.B. Aethoxycarbonyloxymethoxycarbonyl oder 1-Aethoxycarbonyloxyäthoxycarbonyl.

1-Niederalkoxyniederalkoxycarbonyl ist z.B. Methoxymethoxycarbonyl oder 1-Methoxyäthoxycarbonyl.

Bei einer 2-Oxo-1,3-dioxolen-4-ylmethoxycarbonyl-Gruppe, welche in 5-Stellung des Dioxolenringes gegebenenfalls durch Niederalkyl oder Phenyl substituiert ist, handelt es sich vor allem um eine 5-Phenyl- und in erster Linie um eine 5-Methyl-2-oxo-1,3-dioxolen-4-ylmethoxycarbonyl-Gruppe.

Niederalkylamino ist z.B. Methylamino, Aethylamino, n-Propylamino, Isopropylamino oder n-Butylamino, während Diniederalkylamino z.B. Dimethylamino, Diäthylamino, Di-n-propylamino oder Di-n-butylamino bedeutet.

Niederalkanoylamino ist z.B. Formylamino, Acetylamino oder Propionylamino.

Niederalkylenamino weist insbesondere 4 bis 6 Kohlenstoffkettenglieder auf und bedeutet z.B. Pyrrolidino oder Piperidino.

Niederalkoxy ist z.B. Methoxy, Aethoxy, n-Propoxy, Isopropoxy, n-Butoxy oder tert.-Butoxy, während Phenylniederalkoxy z.B. Benzyloxy ist.

Niederalkylthio ist z.B. Methylthio, Aethylthio, n-Propylthio, Isopropylthio oder n-Butylthio.

Niederalkyl ist z.B. Methyl, Aethyl, n-Propyl, Isopropyl, n-Butyl, Isobutyl, sek.-Butyl oder tert.-Butyl.

Aminoniederalkyl ist z.B. 2-Aminoäthyl oder 3-Aminopropyl.

N-Niederalkylaminoniederalkyl ist z.B. 2-Methyl- oder 2-Aethylaminoäthyl, während N,N-Diniederalkylamioniederalkyl z.B. 2-Dimethylaminoäthyl oder 2-Diäthylaminoäthyl bedeutet.

Niederalkenyl ist z.B. Allyl, n-Propenyl oder Isopropenyl.

Bevorzugte, unter physiologischen Bedingungen spaltbare veresterte Carboxylgruppen $R_2$ sind z.B. Phthalidyloxycarbonyl, Niederalkanoyloxymethoxycarbonyl, z.B. Acetoxymethoxycarbonyl oder Pivaloyloxymethoxycarbonyl, und 1-Niederalkoxycarbonyloxyniederalkoxycarbonyl, z.B. 1-Aethoxycarbonyloxyäthoxycarbonyl.

In bevorzugten Methylenaminogruppen der Formel IA als Rest $R_3$ oder als Substituent des Restes $R_4$ ist $X_1$ Wasserstoff, Amino oder Niederalkyl, wie Methyl, und $X_2$ Amino.

Bevorzugte Reste $R_4$ sind z.B. Methyl, Aethyl, Aminomethyl, Aminoäthyl, wie 1- oder 2-Aminoäthyl, Hydroxymethyl und Hydroxyäthyl, wie 1- oder 2-Hydroxyäthyl.

Die in den Verbindungen der Formel I vorhandenen funktionellen Gruppen, wie Hydroxy-, Carboxy- oder Aminogruppen, insbesondere die Hydroxygruppe $R_1$, die Hydroxygruppe als Substituent eines Restes $R_4$ und die Carboxylgruppe $R_2$, sind gegebenenfalls durch Schutzgruppen geschützt, die in der Penem-, Penicillin-, Cephalosporin- und Peptidchemie verwendet werden. Solche Schutzgruppen schützen die betreffenden funktionellen Gruppen vor unerwünschten Kondensationsreaktionen, Substitutionsreaktionen und dergleichen während der Synthese der Verbindung der Formel I aus ihren Vorstufen.

Solche Schutzgruppen sind leicht, d.h. ohne dass unerwünschte Nebenreaktionen stattfinden, beispielsweise solvolytisch, reduktiv oder auch unter physiologischen Bedingungen, abspaltbar.

Schutzgruppen dieser Art sowie ihre Einführung und Abspaltung sind beispielsweise beschrieben in
J.F.W. McOmie, "Protective Groups in Organic Chemistry", Plenum Press, London, New York, 1973,
T.W. Greene, "Protective Groups in Organic Synthesis", Wiley, New York, 1981,

"The Peptides", Vol. I, Schroeder und Luebke, Academic Press, London, New York, 1965 und
Houben-Weyl, "Methoden der Organischen Chemie", Band 15/1, Georg Thieme Verlag, Stuttgart, 1974.

In Verbindungen der Formel (I) kann eine Hydroxygruppe $R_1$, ferner eine im Rest $R_4$ vorhandene Hydroxygruppe, beispielsweise durch Acylreste geschützt sein. Geeignete Acylreste sind z.B. gegebenenfalls durch Halogen substituiertes Niederalkanoyl, z.B. Acetyl oder Trifluoracetyl, gegebenenfalls durch Nitro substituiertes Benzoyl, z.B. Benzoyl, 4-Nitrobenzoyl oder 2,4-Dinitrobenzoyl, gegebenenfalls durch Halogen substituiertes Niederalkoxycarbonyl, z.B. 2-Bromäthoxycarbonyl oder 2,2,2-Trichloräthoxycarbonyl, Niederalkenyloxycarbonyl, z.B. Allyloxycarbonyl, Niederalkenyloxyoxalyl, z.B. Allyloxyoxalyl, oder gegebenenfalls durch Nitro substituiertes Phenylniederalkoxycarbonyl, z.B. 4-Nitrobenzyloxycarbonyl. Weitere geeignete Hydroxyschutzgruppen sind z.B. trisubstituiertes Silyl, wie Triniederalkylsilyl, z.B. Trimethylsilyl oder tert.-Butyl-dimethylsilyl, 2-Halogenniederalkylgruppen, z.B. 2-Chlor-, 2-Brom-, 2-Jod- und 2,2,2-Trichloräthyl, und gegebenenfalls durch Halogen, z.B. Chlor, Niederalkoxy, z.B. Methoxy, und/oder Nitro substituiertes Phenylniederalkyl, wie entsprechendes Benzyl. Bevorzugt als Hydroxyschutzgruppe ist Triniederalkylsilyl, Halogenniederalkoxycarbonyl, Niederalkenyloxyoxalyl und Niederalkenyloxycarbonyl.

Eine Carboxylgruppe $R_2$ ist üblicherweise in veresterter Form geschützt, wobei die Estergruppe unter schonenden Bedingungen, z.B. unter schonend reduktiven, wie hydrogenolytischen, oder schonend solvolytischen, wie acidolytischen oder insbesondere basisch oder neutral hydrolytischen Bedingungen, leicht spaltbar ist. Eine geschützte Carboxylgruppe kann ferner eine leicht in eine andere funktionell abgewandelte Carboxylgruppe, wie in eine andere veresterte Carboxylgruppe umwandelbare, veresterte Carboxylgruppe darstellen.

Solche veresterten Carboxylgruppen enthalten als veresternde Gruppen in erster Linie in 1-Stellung verzweigte oder in 1- oder 2-Stellung geeignet substituierte Niederalkylgruppen. Bevorzugte in veresterter Form vorliegende Carboxylgruppen sind unter anderen Niederalkoxycarbonyl, z.B. Methoxycarbonyl, Aethoxycarbonyl, Isopropoxycarbonyl oder tert.-Butoxycarbonyl, und (Hetero-)Arylmethoxycarbonyl mit 1 bis 3 Arylresten oder einem monocyclischen Heteroarylrest, wobei diese gegebenenfalls z.B. durch Niederalkyl, wie tert.-Niederalkyl, z.B. tert.-Butyl, Halogen, z.B. Chlor, und/oder Nitro mono- oder polysubstituiert sind. Beispiele für solche Gruppen sind gegebenenfalls z.B. wie oben erwähnt, substituiertes Benzyloxycarbonyl, z.B. 4-Nitrobenzyloxycarbonyl, gegebenenfalls, z.B. wie oben erwähnt, substituiertes Diphenylmethoxycarbonyl, z.B. Diphenylmethoxycarbonyl, oder Triphenylmethoxycarbonyl, oder gegebenenfalls, z.B. wie oben erwähnt, substituiertes Picolyloxycarbonyl, z.B. 4-Picolyloxycarbonyl, oder Furfuryloxycarbonyl, wie 2-Furfuryloxycarbonyl. Weitere geeignete Gruppen sind Niederalkanoylmethoxycarbonyl, wie Acetonyloxycarbonyl, Aroylmethoxycarbonyl, worin die Aroylgruppe vorzugsweise gegebenenfalls, z.B. durch Halogen, wie Brom, substituiertes Benzoyl darstellt, z.B. Phenacyloxycarbonyl, Halogenniederalkoxycarbonyl, wie 2-Halogenniederalkoxycarbonyl, z.B. 2,2,2-Trichloräthoxycarbonyl, 2-Chloräthoxycarbonyl, 2-Bromäthoxycarbonyl oder 2-Jodäthoxycarbonyl, oder ω-Halogenniederalkoxycarbonyl, worin Niederalkoxy 4-7 Kohlenstoffatome enthält, z.B. 4-Chlorbutoxycarbonyl, Phthalimidomethoxycarbonyl, Niederalkenyloxycarbonyl, z.B. Allyloxycarbonyl, oder in 2-Stellung durch Niederalkylsulfonyl, Cyano oder trisubstituiertes Silyl, wie Triniederalkylsilyl oder Triphenylsilyl, substituiertes Aethoxycarbonyl, z.B. 2-Methylsulfonyläthoxycarbonyl, 2-Cyanoäthoxycarbonyl, 2-Trimethylsilyläthoxycarbonyl oder 2-(Di-n-butyl-methyl-silyl)-äthoxycarbonyl.

Weitere in veresterter Form vorliegende geschützte Carboxylgruppen sind entsprechende organische Silyloxycarbonyl-, ferner entsprechende organische Stannyloxycarbonylgruppen. In diesen enthält das Silicium- bzw. Zinnatom vorzugsweise Niederalkyl, insbesondere Methyl oder Aethyl, ferner Niederalkoxy, z.B. Methoxy, als Substituenten. Geeignete Silyl- bzw. Stannylgruppen sind in erster Linie Triniederalkylsilyl, insbesondere Trimethylsilyl oder Dimethyl-tert.-butylsilyl, oder entsprechend substituierte Stannylgruppen, z.B. Tri-n-butylstannyl.

Bevorzugte geschützte Carboxylgruppen $R_2'$ sind die 4-Nitrobenzyloxycarbonyl-, Niederalkenyloxycarbonyl-, insbesondere Allyloxycarbonyl-, und die in 2-Stellung durch Niederalkylsulfonyl, Cyano oder Triniederalkylsilyl, z.B. Trimethylsilyl oder Di-n-butyl-methylsilyl, substituierte Aethoxycarbonylgruppe.

Eine geschützte Aminogruppe $R_3$ oder geschütztes Amino als Substituent im Rest $R_4$ kann beispielsweise in Form einer leicht spaltbaren Acylamino-, Acylimino-, verätherten Mercaptoamino-, Silyl- oder Stannylaminogruppe oder als Enamino-, Nitro-oder Azidogruppe vorliegen.

In einer entsprechenden Acylaminogruppe ist Acyl beispielsweise der Acylrest einer organischen Säure mit z.B. bis zu 18 Kohlenstoffatomen, insbesondere einer z.B. durch Halogen oder Phenyl, substituierten Alkancarbonsäure oder gegebenenfalls, z.B. durch Halogen, Niederalkoxy oder Nitro, substituierten Benzoesäure, oder eines Kohlensäurehalbesters. Solche Acylgruppen sind beispielsweise Halogenniederalkanoyl, wie 2-Halogenacetyl, insbesondere 2-Fluor-, 2-Brom-, 2-Jod-, 2,2,2-Trifluor- oder 2,2,2-Trichloracetyl, gegebenenfalls substituiertes Benzoyl, z.B. Benzoyl, Halogenbenzoyl, wie 4-Chlorbenzoyl, Niederalkoxybenzoyl, wie 4-Methoxybenzoyl, oder Nitrobenzoyl, wie 4-Nitrobenzoyl. Insbesondere ist auch Niederalkenyloxycarbonyl, z.B. Allyloxycarbonyl, oder gegebenenfalls in 1- oder 2-Stellung substituiertes Niederalkoxycarbonyl geeignet, wie Niederalkoxycarbonyl, z.B. Methoxy- oder Aethoxycarbonyl, gegebenenfalls substituiertes Benzyloxycarbonyl, z.B. Benzyloxycarbonyl oder 4-Nitrobenzyloxycarbonyl, Aroylmethoxycarbonyl, worin die Aroylgruppe vorzugsweise gegebenenfalls, z.B. durch Halogen, wie Brom, substituiertes Benzoyl darstellt, z.B. Phenacyloxycarbonyl, 2-Halogenniederalkoxycarbonyl, z.B. 2,2,2-Trichloräthoxycarbonyl, 2-Chloräthoxycarbonyl, 2-Bromäthoxycarbonyl oder 2-Jodäthoxycarbonyl, oder 2-(trisubstituiertes Silyl)-äthoxycarbonyl, wie 2-Triniederalkylsilyläthoxycarbonyl, z.B. 2-Trimethylsilyläthoxycarbonyl oder 2-(Di-n-butylmethyl-silyl)-äthoxycarbonyl, oder 2-Triarylsilyläthoxycarbonyl, wie 2-Triphenylsilyläthoxycarbonyl.

In einer Acyliminogruppe ist Acyl beispielsweise der Acylrest einer organischen Dicarbonsäure mit z.B. bis zu 12 Kohlenstoffatomen, insbesondere einer entsprechenden aromatischen Dicarbonsäure, wie Phthalsäure. Eine solche Gruppe ist in erster Linie Phthalimino.

Eine verätherte Mercaptoaminogruppe ist in erster Linie eine gegebenenfalls durch Niederalkyl, wie Methyl oder tert.-Butyl, Niederalkoxy, wie Methoxy, Halogen, wie Chlor oder Brom, und/oder Nitro substituierte Phenylthioaminogruppe oder eine Pyridylthioaminogruppe. Entsprechende Gruppen sind beispielsweise 2- oder 4-Nitrophenylthioamino oder 2-Pyridylthioamino.

Eine Silyl- oder Stannylaminogruppe ist in erster Linie eine organische Silyl- bzw. Stannylaminogruppe, worin das Silicium- bzw. Zinnatom vorzugsweise Niederalkyl, z.B. Methyl, Aethyl, n-Butyl oder tert.-Butyl, ferner Niederalkoxy, z.B. Methoxy, als Substituenten enthält. Entsprechende Silyl- oder Stannylgruppen sind in erster Linie Triniederalkysilyl, insbesondere Trimethylsilyl, ferner Dimethyl-tert.-butylsilyl, oder entsprechend substituiertes Stannyl, z.B. Tri-n-butylstannyl.

Weitere geschützte Aminogruppen sind z.B. Enaminogruppen, die an der Doppelbindung in 2-Stellung einen elektronenziehenden Substituenten, beispielsweise eine Carbonylgruppe, enthalten. Schutzgruppen dieser Art sind beispielsweise 1-Acyl-niederalk-1-en-2-yl-reste, worin Acyl z.B. der entsprechende Rest einer Niederalkancarbonsäure, z.B. Essigsäure, einer gegebenenfalls, z.B. durch Niederalkyl, wie Methyl oder tert.-Butyl, Niederalkoxy, wie Methoxy, Halogen, wie Chlor, und/oder Nitro substituierten Benzoesäure, oder insbesondere eines Kohlensäurehalbesters, wie eines Kohlensäure-niederalkylhalbesters,

z.B. -methylhalbesters oder -äthylhalbesters, und Niederalk-1-en insbesondere 1-Propen bedeutet. Entsprechende Schutzgruppen sind in erster Linie 1-Niederalkanoylprop-1-en-2-yl, z.B. 1-Acetyl-prop-1-en-2-yl, oder 1-Niederalkoxycarbonyl-prop-1-en-2-yl, z.B. 1-Aethoxy-carbonyl-prop-1-en-2-yl.

Bevorzugte geschützte Aminogruppen sind z.B. Azido, Phthalimido, Nitro, Niederalkenyloxycarbonylamino, z.B. Allyloxycarbonylamino, und gegebenenfalls durch Nitro substituiertes Benzyloxycarbonylamino.

Salze von erfindungsgemässen Verbindungen sind in erster Linie pharmazeutisch annehmbare, nichttoxische Salze von Verbindungen der Formel I. Solche Salze werden beispielsweise von den in Verbindungen der Formel I vorhandenen sauren Gruppen, z.B. Carboxyl- und Sulfogruppen, gebildet und sind in erster Linie Metall- oder Ammoniumsalze, wie Alkalimetall- und Erdalkalimetall-, z.B. Natrium-, Kalium-, Magnesium- oder Kalziumsalze, sowie Ammoniumsalze mit Ammoniak oder geeigneten organischen Aminen, wie Niederalkylaminen, z.B. Triäthylamin, Hydroxyniederalkylaminen, z.B. 2-Hydroxyäthylamin, Bis-(2-hydroxyäthyl)-amin oder Tris-(2-hydroxyäthyl)-amin, basischen aliphatischen Estern von Carbonsäuren, z.B. 4-Aminobenzoe-säure-2-diäthylaminoäthylester, Niederalkylenaminen, z.B. 1-Aethylpiperidin, Cycloalkylaminen, z.B. Dicyclohexylamin, oder Benzylaminen, z.B. N,N'-Dibenzyläthylendiamin, Dibenzylamin oder N-Benzyl-ß-phenäthylamin. Verbindungen der Formel I mit einer basischen Gruppe, z.B. mit einer Aminogruppe, können Säureadditionssalze, z.B. mit anorganischen Säuren, wie Salzsäure, Schwefelsäure oder Phosphorsäure, oder mit geeigneten organischen Carbon- oder Sulfonsäure, z.B. Essigsäure, Bernsteinsäure, Fumarsäure, Maleinsäure, Weinsäure, Oxalsäure, Zitronensäure, Benzoesäure, Mandelsäure, Aepfelsäure, Ascorbinsäure, Methansulfonsäure oder 4-Toluolsulfonsäure bilden. Verbindungen der Formel I mit einer sauren und mit einer basischen Gruppe können auch in Form von inneren Salze, d.h. in zwitterionischer Form, vorliegen.

Zur Isolierung oder Reinigung können auch pharmazeutisch ungeeignete Salze Verwendung finden. Zur therapeutischen Anwendung gelangen nur die pharmazeutisch annehmbaren, nicht-toxischen Salze, die deshalb bevorzugt sind.

Die Penem-Verbindungen der Formel I besitzen am 6-Substituenten $-CH(R_1)(CH_3)$, am Methin-Kohlenstoffatom $-CH(R_3)(R_4)$ und gegebenenfalls im Substituenten $R_4$ Chiralitätszentren, die jeweils in der R-, in der S- oder in der racemischen R,S-Konfiguration vorliegen können. In bevorzugten Verbindungen der Formel I besitzt der Rest $-CH(R_1)(CH_3)$ die R-Konfiguration.

Reste der Formel IA, welche am α-Atom des Substituenten $X_1$ oder des Substituenten $X_2$ ein Wasserstoffatom besitzen, z.B. Reste der Formel (IA), worin $X_1$ Amino, Niederalkylamino, Nitroamino, Hydrazino, Anilino oder gegebenenfalls substituiertes Niederalkyl ist und/oder $X_2$ Amino, Niederalkylamino, Hydrazino oder Anilino ist, können auch in einer der tautomeren Formen

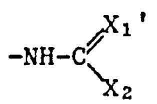 oder 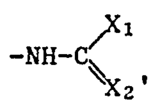 ,

(IB) (IC)

worin $X_1$' bzw. $X_2$' entsprechendes substituiertes oder unsubstituiertes Methylen oder Imino ist, vorliegen.

Die Verbindungen der Formel I weisen wertvolle pharmakologische Eigenschaften auf oder können als Zwischenprodukte zur Herstellung von solchen Verbindungen mit wertvollen pharmakologischen Eigenschaften verwendet werden. Verbindungen der Formel I, worin $R_1$ Hydroxy ist, $R_2$ Carboxyl oder eine unter physiologischen Bedingungen spaltbare veresterte Carboxylgruppe bedeutet, $R_3$ Amino, Niederalkylamino, Diniederalkylamino, Niederalkanoylamino oder wie angegeben substituiertes Methylenamino ist und $R_4$ gegebenenfalls durch Amino, Niederalkylamino, Diniederalkylamino, wie angegeben substituiertes Methylenamino oder Hydroxy substituiertes Methyl oder Aethyl

darstellt, und pharmakologisch annehmbare Salze von solchen Verbindungen mit salzbildenden Gruppen, haben antibakterielle Wirkungen. Beispielsweise sind sie in vitro gegen grampositive und gramnegative Kokken, z.B. Staphylococcus aureus, Streptococcus pyogenes, Streptococcus pneumoniae, Streptococcus faecalis, Neisseria meningitidis und Neisseria gonorrhoeae, gegen Enterobakterien, z.B. Escherichia coli, Proteus mirabilis und Klebsiella pneumoniae, gegen Haemophilus influenzae, Pseudomonas aeruginosa und Anaerobier, z.B. Bacteroides fragilis und Clostridium perfringens in minimalen Konzentrationen von ca. 0,01 bis ca. 64 µg/ml wirksam. In vivo, bei der systemischen Infektion der Maus, z.B. durch Staphylococcus aureus, Escherichia coli oder Streptococcus pyogenes, ergeben sich bei subkutaner oder oraler Applikation $ED_{50}$-Werte von ca. 0,1 bis ca. 100 mg/kg.

Ueberraschenderweise weisen die erfindungsgemässen Penem-Verbindungen gegenüber strukturverwandten und vorbekannten Penemen eine überlegene Wirksamkeit auf. Dies wird deutlich beim Vergleich der MIC-Daten (minimum inhibitory concentrations, in vitro gemessen) von (5R,6S)-2-[(2R,S)-2-Amino-but-1-yl)-6-[(1R)-1-hydroxyäthyl]-2-penem-3-carbonsäure (Verbindung A) mit bekannten homologen Penemen, die in Position 2 des Penem-Ringes einen 4-Aminobutyl- bzw. 1-Aminobutyl-Substituenten tragen, nämlich (5R,6S)-2-(4-Aminobutyl)-6-hydroxymethyl-2-penem-3-carbonsäure (Europäische Patentanmeldung Nr. 82113, Verbindung B) und (5R,6S)-2-(1-Aminobutyl)-6-hydroxymethyl-2-penem-3-carbonsäure (Europäische Patentanmeldung Nr. 125 208, Verbindung C).
Die Ergebnisse sind in der folgenden Tabelle 1 zusammengefasst.

Tabelle 1: Antibiotische Wirksamkeit in vitro

| Mikroorganismus | in vitro MIC (µg/ml) Verbindung A | Verbindung B | Verbindung C |
|---|---|---|---|
| Staphylococcus aureus 10 B | 0,02 | 0,5 | 0,05 |
| Staphylococcus aureus 2999i+p+ | 0,05 | 0,5 | 0,05 |
| Staphylococcus aureus A 124 | 0,1 | n.b. | 0,5 |
| Staphylococcus aureus Wood 46 | 0,02 | n.b. | 0,05 |
| Streptococcus pyogenes Aronson 1129 | 0,05 | 0,5 | 1 |
| Streptococcus pneumoniae III/84 | 0,05 | 0,5 | 0,05 |
| Neisseria meningitidis 1316 | 1 | n.b. | 1 |
| Neisseria gonorrhoeae 1317/4 | 0,5 | n.b. | 2 |
| Haemophilus influenzae 24 | 16 | n.b. | 32 |
| Escherichia coli 205 | 1 | 4 | 64 |
| Escherichia coli 205 R + TEM | 2 | 8 | 64 |
| Escherichia coli 16 | 1 | 4 | 64 |
| Escherichia coli 2018 | 0,5 | n.b. | 32 |
| Escherichia coli UB 1005 | 2 | 4 | 64 |
| Escherichia coli DC 2 | 16 | 8 | 64 |
| Escherichia coli B-1385 | 1 | n.b. | 64 |
| Klebsiella pneumoniae 327 | 1 | 4 | 32 |
| Serratia marcescens 344 | 8 | 16 | >64 |
| Enterobacter cloacae P 99 | 0,5 | 4 | 64 |
| Enterobacter cloacae ATCC 13074 | 2 | 16 | >64 |
| Proteus mirabilis 774 | 2 | 16 | >64 |
| Proteus morganii 2359 | 8 | 16 | >64 |
| Pseudomonas aeruginosa ATCC 12055 | 32 | 8 | >64 |
| Pseudomonas aeruginosa K 799/61 | 4 | 4 | 64 |
| Pseudomonas aeruginosa 143738R | 64 | n.b. | >64 |
| Clostridium perfringens 194 AN | 0,2 | 4 | 64 |
| Bacteroides fragilis L 01 | 0,5 | 0,5 | 16 |

(n.b.: nicht bestimmt)

Die neuen Verbindungen können daher als oral oder parenteral applizierbare antibakterielle Antibiotika, z.B. in Form von entsprechenden pharmazeutischen Präparaten, zur Behandlung von Infektionen Verwendung finden.

Verbindungen der Formel I, worin mindestens eine der vorhandenen funktionellen Gruppen in geschützter Form vorliegt, wobei eine geschützte Carboxylgruppe von einer physiologisch spaltbaren veresterten Carboxylgruppe verschieden ist, können als Zwischenprodukte zur Herstellung der oben genannten pharmakologisch wirksamen Verbindungen der Formel I verwendet werden.

Die Erfindung betrifft vor allem Verbindungen der Formel I, worin $R_1$ Hydroxy oder geschütztes Hydroxy ist; $R_2$ Carboxyl, unter physiologischen Bedingungen spaltbares verestertes Carboxyl oder geschütztes Carboxyl $R_2'$ ist, $R_3$ Amino, Niederalkylamino, Diniederalkylamino, Niederalkanoylamino, eine Gruppe der Formel $-N=C(X_1,X_2)$, worin $X_1$ Wasserstoff, Amino, Niederalkylamino, Diniederalkylamino, Niederalkylenamino, Nitroamino, Hydrazino, Anilino, Niederalkoxy, Phenylniederalkoxy, Niederalkylthio, Niederalkyl, Aminoniederalkyl, N-Niederalkylaminoniederalkyl, N,N-Diniederalkylaminoniederalkyl, Niederalkenyl, Phenyl oder über ein Ringkohlenstoffatom gebundenes monocyclisches 5- oder 6-gliedriges Heteroaryl mit 1 oder 2 Stickstoffatomen und/oder einem Sauerstoff-oder Schwefelatom, ist, und $X_2$ Amino, Niederalkylamino, Diniederalkylamino, Niederalkylenamino, Hydrazino, Anilino, Niederalkoxy, Phenylniederalkoxy oder Niederalkylthio ist, oder geschütztes Amino darstellt; $R_4$ Methyl, Aethyl oder durch Amino, Niederalkylamino, Diniederalkylamino, eine Gruppe der Formel $-N=C(X_1,X_2)$, worin $X_1$ und $X_2$ die bei der Definition von $R_3$ angegebenen Bedeutungen haben, geschütztes Amino, Hydroxy oder geschütztes Hydroxy substituiertes Methyl oder Aethyl, bedeutet, und m eine ganze Zahl von 1 bis 3 ist, optische Isomere von Verbindungen der Formel I, Mischungen dieser optischen Isomere und Salze von solchen Verbindungen der Formel I, die eine salzbildende Gruppe aufweisen.

Die Erfindung betrifft hauptsächlich Verbindungen der Formel I, worin $R_1$ Hydroxy ist, $R_2$ Carboxyl oder unter physiologischen Bedingungen spaltbares verestertes Carboxyl, z.B. Niederalkanoyloxymethoxycarbonyl oder 1-Niederalkoxycarbonyloxyniederalkoxycarbonyl, bedeutet, $R_3$ Amino, Niederalkylamino, Diniederalkylamino, Niederalkanoylamino oder eine Gruppe der Formel $-N=C(X_1,X_2)$, worin $X_1$ Wasserstoff, Niederalkyl oder Amino und $X_2$ Amino ist, darstellt, $R_4$ Methyl, Aethyl oder durch Amino, Niederalkylamino oder Hydroxy substituiertes Methyl oder Aethyl ist, und m 1 oder 2 bedeutet, optische Isomere von Verbindungen der Formel I, Mischungen dieser optischen Isomere und Salze von solchen Verbindungen der Formel I, die eine salzbildende Gruppe aufweisen.

Die Erfindung betrifft in erster Linie Verbindungen der Formel I, worin $R_1$ Hydroxy ist, $R_2$ Carboxyl oder unter physiologischen Bedingungen spaltbares verestertes Carboxyl, z.B. Niederalkanoyloxymethoxycarbonyl oder 1-Niederalkoxycarbonyloxyniederalkoxycarbonyl, darstellt, $R_3$ Amino ist, $R_4$ Methyl oder Aethyl bedeutet und m 1 oder 2 ist, optische Isomere von Verbindungen der Formel I, Mischungen dieser optischen Isomere und Salze von solchen Verbindungen der Formel I, die eine salzbildende Gruppe aufweisen.

Die Erfindung betrifft insbesondere die in den Beispielen genannten Verbindungen der Formel I, ihre optischen Isomere und ihre Salze.

Die Verbindungen der vorliegenden Erfindung können nach an sich bekannten Verfahren hergestellt werden.

Die neuen Verbindungen werden z.B. hergestellt, indem man

a) eine Ylid-Verbindung der Formel

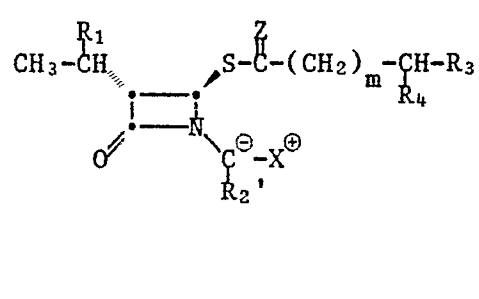

(II) ,

worin $R_1$, $R_3$, $R_4$ und m die unter Formel I angegebenen Bedeutungen haben, $R_2'$ geschütztes Carboxyl ist, Z Sauerstoff oder Schwefel bedeutet und $X^{\oplus}$ entweder eine dreifach substituierte Phosphoniogruppe oder eine zweifach veresterte Phosphonogruppe zusammen mit einem Kation darstellt, ringschliesst, oder

b) eine Verbindung der Formel

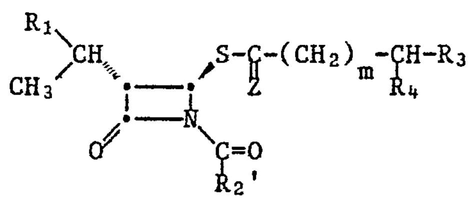

(III) ,

worin $R_1$, $R_3$, $R_4$ und m die unter Formel I angegebenen Bedeutungen haben, $R_2'$ geschütztes Carboxyl ist und Z Sauerstoff oder Schwefel ist, mit einer organischen Verbindung des dreiwertigen Phosphors behandelt, oder

c) in einer Verbindung der Formel

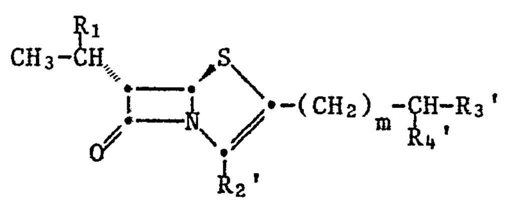

(IV) ,

worin $R_1$ und m die unter Formel I angegebenen Bedeutungen haben, $R_2'$ eine geschützte Carboxylgruppe ist, und $R_3'$ eine durch den Rest $R_3$ ersetzbare Gruppe oder $R_3$ ist und $R_4'$ eine in den Rest $R_4$ überführbare Gruppe oder $R_4$ ist, mit der Massgabe, dass mindestens eine der Gruppen $R_3'$ und $R_4'$ von $R_3$ bzw. $R_4$ verschieden ist, die Gruppe $R_3'$ durch den Rest $R_3$ ersetzt und/oder die Gruppe $R_4'$ in den Rest $R_4$ überführt, oder

d) in einer Verbindung der Formel

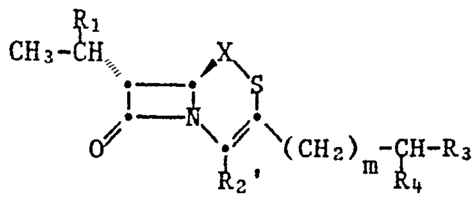

(V) ,

worin $R_1$, $R_3$, $R_4$ und m die unter Formel I angegebenen Bedeutungen haben, $R_2'$ eine geschützte Carboxylgruppe darstellt und X für die Gruppe -S- oder für die Gruppe $-SO_2-$ steht, den Rest X abspaltet, und, wenn erwünscht oder notwendig, in einer erhältlichen Verbindung der Formel I eine geschützte Hydroxygruppe $R_1$ oder geschütztes Hydroxy im Rest $R_4$ in die freie Hydroxygruppe überführt, und/oder, wenn erwünscht, in einer erhältlichen Verbindung der Formel I eine geschützte Carboxylgruppe $R_2'$ in die freie Carboxylgruppe, in eine unter physiologischen Bedingungen spaltbare veresterte Carboxylgruppe oder in eine andere geschützte Carboxylgruppe $R_2'$ oder eine freie Carboxylgruppe $R_2$ in eine unter physiologischen Bedingungen spaltbare veresterte Carboxylgruppe überführt, und/oder, wenn erwünscht, eine geschützte Aminogruppe $R_3$ oder geschütztes Amino im Rest $R_4$ in die freie Aminogruppe oder eine freie Aminogruppe $R_3$ oder freies Amino im Rest $R_4$ in eine substituierte Aminogruppe überführt, und/oder, wenn erwünscht, ein erhältliches Gemisch von isomeren Verbindungen der Formel I in die einzelnen Isomere auftrennt, und/oder, wenn erwünscht, eine erhältliche Verbindung mit salzbildender Gruppe in ein Salz oder ein erhältliches Salz in die freie Verbindung oder in ein anderes Salz überführt.

In den Ausgangsverbindungen der Formeln II-V sind funktionelle Gruppen, wie z.B. eine freie Hydroxygruppe $R_1$ und z.B. eine freie Aminogruppe $R_3$, vorzugsweise durch konventionelle Schutzgruppen, z.B. durch eine der oben genannten, geschützt.

a) Cyclisierung der Verbindung der Formel II

Die Gruppe $X^{\oplus}$ im Ausgangsmaterial der Formel II ist eine der bei Wittig-Kondensationsreaktionen gebräuchlichen Phosphonio- oder Phosphonogruppen, insbesondere eine Triaryl-, z.B. Triphenyl-, oder Triniederalkyl-, z.B. Tri-n-butylphosphoniogruppe, oder eine durch Niederalkyl, z.B. Aethyl, zweifach veresterte Phosphonogruppe, wobei das Symbol $X^{\oplus}$ für den Fall der Phosphonogruppe zusätzlich das Kation einer starken Base, insbesondere ein geeignetes Metall-, wie Alkalimetallion, z.B. das Lithium-, Natrium- oder Kaliumion, umfasst. Bevorzugt als Gruppe $X^{\oplus}$ sind einerseits Triphenylphosphonio und andererseits Diäthylphosphono zusammen mit einem Alkalimetall-, z.B. dem Natriumion.

In Phosphonio-Verbindungen der Formel II wird die negative Ladung durch die positiv geladene Phosphoniogruppe neutralisiert. In Phosphono-Verbindungen der Formel II wird die negative Ladung durch das Kation einer starken Base neutralisiert, das je nach Herstellungsweise des Phosphono-Ausgangsmaterials z.B. ein Alkalimetall-, z.B. Natrium-, Lithium- oder Kaliumion, sein kann. Die Phosphono-Ausgangsstoffe werden daher als Salze in die Reaktion eingesetzt.

Der Ringschluss kann spontan, d.h. bei der Herstellung der Ausgangsstoffe, oder durch Erwärmen, z.B. in einem Temperaturbereich von etwa 30°C bis 160°C, vorzugsweise von etwa 50°C bis etwa 100°C, erfolgen. Die Reaktion wird vorzugsweise in einem geeigneten inerten Lösungsmittel, wie in einem aliphatischen, cycloaliphatischen oder aromatischen Kohlenwasserstoff, z.B. Cyclohexan, Benzol oder Toluol, einem halogenierten Kohlenwasserstoff, z.B. Methylenchlorid, einem Aether, z.B. Diäthyläther, einem cyclischen Aether, z.B. Dioxan oder Tetrahydrofuran, einem Carbonsäureamid, z.B. Dimethylformamid, einem Diniederalkylsulfoxid, z.B. Dimethylsulfoxid, oder einem Niederalkanol, z.B. Aethanol, oder in einem Gemisch davon, und, falls notwendig, in einer Inertgas-, z.B. Stickstoffatmosphäre, durchgeführt.

b. Cyclisierung der Verbindung der Formel III

Eine organische Verbindung des dreiwertigen Phosphors leitet sich z.B. von phosphoriger Säure ab und ist insbesondere ein Ester derselben mit einem Niederalkanol, z.B. Methanol oder Aethanol, und/oder einer gegebenenfalls substituierten aromatischen Hydroxyverbindung, z.B. Phenol oder Brenzcatechin, oder ein Amidester derselben der Formel $P(OR_a)-N(R_b)_2$, worin $R_a$ und $R_b$ unabhängig voneinander Niederalkyl, z.B. Methyl, oder Aryl, z.B. Phenyl, bedeuten. Bevorzugte Verbindungen des dreiwertigen Phosphors sind Trialkylphosphite, z.B. Trimethylphosphit oder Triäthylphosphit.

Die Reaktion wird vorzugsweise in einem inerten Lösungsmittel, wie einem aromatischen Kohlenwasserstoff, z.B. Benzol oder Toluol, einem Aether, z.B. Dioxan oder Tetrahydrofuran, oder einem halogenierten Kohlenwasserstoff, z.B. Methylenchlorid oder Chloroform, bei einer Temperatur von etwa 20° bis 140°C, bevorzugt von etwa 40° bis etwa 110°C, durchgeführt, wobei man 1 Moläquivalent einer Verbindung der Formel III mit 2 Moläquivalent der Phosphorverbindung umsetzt. Vorzugsweise legt man die Verbindung der Formel III in einem inerten Lösungsmittel vor und tropft die Phosphorverbindung, vorzugsweise in dem gleichen inerten Lösungsmittel gelöst, über einen längeren Zeitraum, z.B. während eines Zeitraums von 2 bis 4 Stunden, hinzu.

In einer bevorzugten Ausführungsform des Verfahrens stellt man das Ausgangsmaterial der Formel III wie weiter unten angegeben her und setzt es ohne Isolierung aus dem Reaktionsgemisch mit der organischen Verbindung des dreiwertigen Phosphors um, wobei die Endprodukte der Formel I entstehen.

c) Ersetzung des Restes $R_3$ und/oder $R_4$

Eine durch den Rest $R_3$ ersetzbare Gruppe $R_3'$ ist beispielsweise eine entsprechende, durch nucleophile Reaktion ersetzbare Gruppe. Solche Gruppen $R_3'$ sind insbesondere veresterte Hydroxygruppen, wie durch eine Halogenwasserstoffsäure, eine Diniederalkyl- oder Diarylphosphorsäure, eine gegebenenfalls durch Oxo oder Halogen substituierte

Niederalkancarbonsäure, eine gegebenenfalls durch Halogen substituierte Niederalkan- oder eine gegebenenfalls durch Halogen oder Niederalkyl substituierte Benzolsulfonsäure veresterte Hydroxygruppen. Entsprechende in den Rest $R_4$ überführbare Gruppen $R_4'$ sind beispielsweise durch eine der genannten veresterten Hydroxygruppen substituiertes Methyl oder Aethyl. Solche veresterten Hydroxygruppen sind z.B. Halogen, wie Chlor, Brom oder Jod, Diniederalkyl- oder Diarylphosphoryloxy, z.B. Dimethyl-, Diäthyl- oder Diphenylphosphoryloxy, gegebenenfalls durch Halogen oder Oxo substituiertes Niederalkanoyloxy, z.B. Formyloxy, Acetyloxy oder Acetacetyloxy, gegebenenfalls durch Halogen substituiertes Niederalkansulfonyloxy, z.B. Methansulfonyloxy oder Trifluormethansulfonyloxy, oder gegebenenfalls durch Halogen oder Niederalkyl substituiertes Benzolsulfonyloxy, z.B. Benzolsulfonyloxy, 4-Chlor-, 4-Brom- oder 4-Methylbenzolsulfonyloxy.

Dabei wird beispielsweise eine veresterte Hydroxygruppe $R_3'$ durch eine Aminogruppe oder durch eine, wie bei der Definition von $R_3$ angegeben, substituierte Aminogruppe $R_3$ und/oder eine veresterte Hydroxygruppe im Rest $R_4'$ durch eine Aminogruppe, durch eine, wie bei der Definition von $R_4$ angegeben, substituierte Aminogruppe oder durch eine Hydroxygruppe ersetzt.

Die Umsetzung erfolgt mit einer Verbindung der Formel Z'-H, worin Z' im Falle der Substitution des Restes $R_3'$ für Amino, Niederalkanoylamino, durch Niederalkyl substituiertes Amino, substituiertes Amino oder geschütztes Amino und im Falle der Substitution am Rest $R_4'$ für Amino, durch Niederalkyl substituiertes Amino, substituiertes Methylenamino, geschütztes Amino, Hydroxy oder geschütztes Hydroxy steht, oder einem Salz, wie einem Alkalimetallsalz, z.B. dem Natrium- oder Kaliumsalz davon, d.h. zur Herstellung entsprechender Aminoverbindungen mit Stickstoffwasserstoffsäure oder mit Phthalimid oder insbesondere mit Alkalimetallsalzen, z.B. dem Natrium- oder Kaliumsalz davon, ferner mit Ammoniak, Niederalkyl- und Diniederalkylaminen, Amidinen, Guanidinen und dergleichen, welche gegebenenfalls in geschützter Form vorliegen, wobei an der reagierenden

Aminogruppe aber zumindest ein Wasserstoffatom vorhanden sein muss, und zur Herstellung entsprechender Hydroxyverbindungen z.B. mit dem Hydroxid oder Carbonat eines Alkalimetalls, z.B. Natriumhydroxid, Natriumcarbonat oder Kaliumcarbonat, ferner mit Alkalimetall-, wie Natriumsalzen, entsprechender Kohlensäurehalbester, wie z.B. Kohlensäureallylester oder Kohlensäurehalogenalkylester, und dergleichen. Die Reaktion wird in bekannter Weise in einem inerten Lösungsmittel, wie z.B. einem Niederalkanol, Dimethylformamid, einem cyclischen Aether, z.B. Dioxan, in Dimethylsulfoxid, in Wasser oder in Gemischen davon, bei Raumtemperatur oder etwas erhöhter oder erniedrigter Temperatur, z.B. bei etwa 0° bis 40°C, insbesondere bei Raumtemperatur, durchgeführt.

Die Ausgangsverbindungen der Formel IV können in analoger Weise, wie unter Verfahren a) beschrieben, durch Ringschluss des Phosphorans der Formel

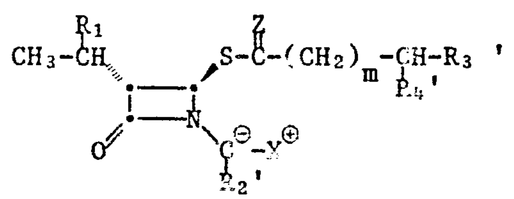

(VI) ,

worin Z und $X^{\oplus}$ die unter Formel II angegebenen Bedeutungen haben, hergestellt werden.

d) <u>Eliminierung des Restes X aus Verbindungen der Formel V</u>

Bei den Verbindungen der Formel V handelt es sich um entsprechend substituierte 2-Thia-3-cephem-4-carbonsäuren oder insbesondere um 2-Thia-3-cephem-4-carbonsäure-1,1-dioxide.

Die Eliminierung von Schwefel aus einer 2-Thia-3-cephem-4-carbonsäure der Formel V (X=S) erfolgt beispielsweise mit einer organischen Verbindung des dreiwertigen Phosphors, wie z.B. einer der unter Verfahren b) genannten Verbindungen. Geeignete Verbindungen des dreiwertigen Phosphors sind z.B. Triniederalkylphosphite, z.B. Triäthylphosphit, und insbesondere Triphenylphosphin. Die Eliminierung wird bei Raumtemperatur oder leicht erhöhter Temperatur, z.B. bei etwa 20° bis etwa 60°C, in einem inerten Lösungsmittel oder Lösungsmittelgemisch, z.B. in einem Kohlenwasserstoff, z.B. Benzol, einem chlorierten Kohlenwasserstoff, z.B. Chloroform, oder einem Niederalkanon, z.B. Aceton, wenn nötig in einer Inertgas-, wie Stickstoffatmosphäre, durchgeführt.

Die Eliminierung von Schwefeldioxid aus einer Verbindung der Formel V, worin X für $SO_2$ steht, erfolgt beispielsweise in einer diese Verbindung erhaltenden Lösung durch mildes bis mässiges Erwärmen, z.B. auf etwa 30° bis etwa 80°C, wobei als Lösungsmittel insbesondere inerte Lösungsmittel, wie z.B. gegebenenfalls chlorierte Kohlenwasserstoffe, z.B. Chloroform, aliphatische Aether oder aromatische Kohlenwasserstoffe, z.B. Benzol, in Frage kommen.

Bevorzugt werden solche Ausgangsmaterialien der Formeln II-VI verwendet, die zu den eingangs als besonders bevorzugt genannten Verbindungen der Formel I führen.

Erhältliche Verbindungen der Formel I können in an sich bekannter Weise im Rahmen der Definitionen in andere Verbindungen der Formel I überführt werden.

In einer erhältlichen Verbindung der Formel I, worin eine oder mehrere funktionelle Gruppen geschützt sind, können diese, z.B. geschützte Carboxyl-, Hydroxy- und/oder Aminogruppen, in an sich bekannter Weise mittels Solvolyse, insbesondere Hydrolyse, Alkoholyse oder Acidolyse, oder mittels Reduktion, insbesondere Hydrogenolyse oder chemische Reduktion, gegebenenfalls stufenweise oder gleichzeitig freigesetzt werden.

In einer verfahrensgemäss erhältlichen Verbindung der Formel I, worin $R_2$ eine geschützte Carboxylgruppe bedeutet, kann die geschützte Carboxylgruppe in an sich bekannter Weise freigesetzt werden. So kann man tert.-Niederalkoxycarbonyl oder in 2-Stellung durch eine trisubstituierte Silylgruppe oder in 1-Stellung durch Niederalkoxy

substituiertes Niederalkoxycarbonyl oder gegebenenfalls substituiertes Diphenylmethoxyarbonyl z.B. durch Behandeln mit einer Carbonsäure, wie Ameisensäure oder Trifluoressigsäure, gegebenenfalls unter Zugabe einer nucleophilen Verbindung, wie Phenol oder Anisol, in freies Carboxyl überführen. Gegebenenfalls substituiertes Benzyloxycarbonyl kann z.B. mittels Hydrogenolyse, d.h. durch Behandeln mit Wasserstoff in Gegenwart eines metallischen Hydrierkatalysators, wie eines Palladiumkatalysators, gespalten werden. Ferner kann man geeignet substituiertes Benzyloxycarbonyl, wie 4-Nitrobenzyloxycarbonyl, auch mittels chemischer Reduktion, z.B. durch Behandeln mit einem Alkalimetall-, z.B. Natriumdithionit, oder mit einem reduzierenden Metall, z.B. Zinn, oder Metallsalz, wie einem Chrom-II-Salz, z.B. Chrom-II-chlorid, üblicherweise in Gegenwart eines Wasserstoff-abgebenden Mittels, das zusammen mit dem Metall nascierenden Wasserstoff zu erzeugen vermag, wie einer geeigneten Carbonsäure, z.B. einer gegebenenfalls, z.B. durch Hydroxy, substituierten Niederalkancarbonsäure, z.B. Essigsäure, Ameisensäure oder Glykolsäure, oder eines Alkohols oder Thiols, wobei man vorzugsweise Wasser zugibt, in freies Carboxyl überführen. Die Abspaltung einer Allylschutzgruppe kann z.B. durch Umsetzung mit einer Verbindung des Palladiums, z.B. Tetrakis-(triphenylphosphin)-palladium, in Gegenwart von Triphenylphosphin und unter Zusatz einer Carbonsäure, z.B. 2-Aethylhexansäure, oder eines Salzes davon erfolgen. Durch Behandeln mit einem reduzierenden Metall oder Metallsalz, wie oben beschrieben, kann man auch 2-Halogenniederalkoxycarbonyl (gegebenenfalls nach Umwandlung einer 2-Bromniederalkoxycarbonylgruppe in eine entsprechende 2-Jodniederalkoxycarbonylgruppe) oder Aroylmethoxycarbonyl in freies Carboxyl umwandeln, während Aroylmethoxycarbonyl ebenfalls durch Behandeln mit einem nucleophilen, vorzugsweise salzbildenden Reagenz, wie Natriumthiophenolat oder Natriumjodid, gespalten werden kann. Substituiertes 2-Silyläthoxycarbonyl kann auch durch Behandeln mit einem das Fluoridanion liefernden Salz der Fluorwasserstoffsäure, wie einem Alkalimetallfluorid, z.B. Natriumfluorid, in Anwesenheit eines makrocyclischen Polyäthers ("Kronenäther") oder mit einem Fluorid einer organischen quaternären Base, wie Tetraniederalkylammoniumfluorid, z.B. Tetrabutylammoniumfluorid, in freies Carboxyl überführt werden. Mit einer organischen Silyl- oder Stannylgruppe, wie Triniederalkylsilyl oder -stannyl, verestertes Carboxyl kann in üblicher Weise solvolytisch, z.B. durch Behandeln mit Wasser oder einem Alkohol, freigesetzt werden. Eine in 2-Stellung durch Niederalkylsulfonyl oder Cyano substituierte Niederalkoxycarbonylgruppe kann z.B. durch Behandeln mit einem basischen Mittel, wie einem Alkalimetall- oder Erdalkalimetallhydroxid oder -carbonat, z.B. Natrium- oder Kaliumhydroxid oder Natrium- oder Kaliumcarbonat, in freies Carboxyl übergeführt werden.

Andererseits können auch Verbindungen der Formel I, worin $R_2$ Carboxy, bedeutet, in Verbindungen der Formel I überführt werden, worin $R_2$ eine geschützte Carboxylgruppe, insbesondere eine veresterte Carboxylgruppe, oder eine unter physiologischen Bedingungen spaltbare veresterte Carboxylgruppe darstellt. So kann man die freie Carboxylgruppe z.B. durch Behandeln mit einer geeigneten Diazoverbindung, wie einem Diazoniederalkan, z.B. Diazomethan, oder einem Phenyldiazoniederalkan, z.B. Diphenyldiazomethan, wenn notwendig, in Gegenwart einer Lewis-Säure, wie z.B. Bortrifluorid, oder durch Umsetzen mit einem zur Veresterung geeigneten Alkohol in Gegenwart eines Veresterungsmittels, wie eines Carbodiimids, z.B. Dicyclohexylcarbodiimid, sowie Carbonyldiimidazol, verestern. Ester können auch durch Umsetzung eines gegebenenfalls in situ hergestellten Salzes der Säure mit einem reaktionsfähigen Ester eines Alkohols und einer starken anorganischen Säure, wie Schwefelsäure, oder einer starken organischen Sulfonsäure, wie 4-Toluolsulfonsäure, hergestellt werden. Ferner können Säurehalogenide, wie Chloride (hergestellt z.B. durch Behandeln mit Oxalylchlorid), aktivierte Ester, (gebildet z.B. mit N-Hydroxystickstoffverbindungen, wie N-Hydroxysuccinimid) oder gemischte Anhydride (erhalten z.B. mit Halogenameisensäure-niederalkylester, wie Chlorameisensäureäthyl- oder Chlorameisensäureisobutylester, oder mit Halogenessigsäurehalogeniden, wie Trichloressigsäurechlorid) durch Umsetzen mit geeigneten Alkoholen, gegebenenfalls in Gegenwart einer Base, wie Pyridin, in eine veresterte Carboxylgruppe übergeführt werden.

In einer Verbindung der Formel I mit einer veresterten Carboxylgruppe $R_2$ kann diese in eine andere veresterte Carboxylgruppe übergeführt werden, z.B. 2-Chloräthoxycarbonyl oder 2-Bromäthoxycarbonyl durch Behandeln mit einem Jodsalz, z.B. Natriumjodid, in 2-Jodäthoxycarbonyl. Ferner kann man in Verbindungen der Formel I, die eine in veresterter Form geschützte Carboxylgruppe enthalten, die Carboxylschutzgruppe wie oben beschrieben abspalten und eine entstandene Verbindung der Formel I mit einer freien Carboxylgruppe oder ein Salz davon durch Umsetzen mit dem reaktionsfähige Ester eines entsprechenden Alkohols in eine Verbindung der Formel I überführen, worin $R_2$ eine unter physiologischen Bedingungen spaltbare veresterte Carboxylgruppe darstellt.

In verfahrensgemäss erhältlichen Verbindungen der Formel I, worin $R_1$ geschütztes Hydroxyl ist und/oder gegebenenfalls der Rest $R_4$ geschütztes Hydroxyl als Substituenten enthält, kann die geschützte Hydroxygruppe in an sich bekannter Weise in die freie Hydroxygruppe überführt werden. Beispielsweise wird eine durch eine geeignete Acylgruppe oder eine organische Silyl- oder Stannylgruppe geschützte Hydroxygruppe wie eine entsprechend geschützte Aminogruppe freigesetzt (s.u.); eine Triniederalkylsilylgruppe z.B. auch mit Tetrabutylammoniumfluorid und Essigsäure (unter diesen Bedingungen werden durch trisubstituiertes Silyläthoxy geschützte Carboxygruppen nicht gespalten). Eine 2-Halogenniederalkylgruppe und eine gegebenenfalls substituierte Benzylgruppe werden reduktiv abgespalten.

In einer erfindungsgemäss erhältlichen Verbindung der Formel I mit einer geschützten Aminogruppe kann diese in an sich bekannter Weise, z.B. je nach Art der Schutzgruppe, vorzugsweise mittels Solvolyse oder Reduktion, in die freie Aminogruppe übergeführt werden. Beispielsweise kann 2-Halogenniederalkoxycarbonylamino (gegebenenfalls nach Umwandlung einer 2-Bromniederalkoxycarbonylaminogruppe in eine 2-Jodniederalkoxycarbonylaminogruppe), Aroylmethoxycarbonylamino oder 4-Nitrobenzyloxycarbonylamino durch Behandeln mit einem geeigneten chemischen Reduktionsmittel, wie Zink in Gegenwart einer

geeigneten Carbonsäure, wie wässriger Essigsäure, oder katalytisch mit Wasserstoff in Gegenwart eines Palladiumkatalysators gespalten werden. Aroylmethoxycarbonylamino kann auch durch Behandeln mit einem nucleophilen, vorzugsweise salzbildenden Reagenz, wie Natriumthiophenolat, und 4-Nitrobenzyloxycarbonylamino auch durch Behandeln mit einem Alkalimetall-, z.B. Natriumdithionit gespalten werden. Gegebenenfalls substituiertes Benzyloxycarbonylamino kann z.B. mittels Hydrogenolyse, d.h. durch Behandeln mit Wasserstoff in Gegenwart eines geeigneten Hydrierkatalysators, wie eines Palladiumkatalysators, und Allyloxycarbonylamino durch Umsetzen mit einer Verbindung des Palladiums, z.B. Tetrakis(triphenylphosphin)palladium, in Gegenwart von Triphenylphosphin und Behandeln mit einer Carbonsäure, z.B. 2-Aethylhexansäure, oder eines Salzes davon, gespalten werden. Eine mit einer organischen Silyl- oder Stannylgruppe geschützte Aminogruppe kann z.B. mittels Hydrolyse oder Alkoholyse, eine durch 2-Halogenniederalkanoyl, z.B. 2-Chloracetyl, geschützte Aminogruppe durch Behandeln mit Thioharnstoff in Gegenwart einer Base oder mit einem Thiolatsalz, wie einem Alkalimetallthiolat, des Thioharnstoffs und anschliessender Solvolyse, wie Alkoholyse oder Hydrolyse des entstandenen Kondensationsproduktes freigesetzt werden. Eine durch 2-substituiertes Silyläthoxycarbonyl geschützte Aminogruppe kann durch Behandeln mit einem Fluoridanion liefernden Salz der Fluorwasserstoffsäure, wie einem Alkalimetallfluorid, z.B. Natriumfluorid, in Anwesenheit eines makrocyclischen Polyäthers ("Kronenäther") oder mit einem Fluorid einer organischen quaternären Base, wie Tetraniederalkylammoniumfluorid, z.B. Tetraäthylammoniumfluorid, in die freie Aminogruppe übergeführt werden. Eine in Form einer Azido- oder Nitrogruppe geschützte Aminogruppe wird z.B. durch Reduktion in freies Amino überführt, beispielsweise durch katalytische Hydrierung mit Wasserstoff in Gegenwart eines Hydrierkatalysators wie Platinoxid, Palladium oder Raney-Nickel, oder durch Behandeln mit Zink in Gegenwart einer Säure, wie Essigsäure. Eine in Form einer Phthalimidogruppe geschützte Aminogruppe kann durch Umsetzen mit Hydrazin in die freie Aminogruppe überführt werden. Weiterhin kann eine Arylthioaminogruppe durch Behandeln mit einem nucleophilen Reagenz, wie schwefliger Säure, in Amino umgewandelt werden.

Ferner kann man eine freie Aminogruppe $R_3$ oder Amino als Substituent des Restes $R_4$ in an sich bekannter Weise in eine substituierte Aminogruppe überführen. So kann man beispielsweise Amino $R_3$ durch Umsetzen mit einem entsprechenden Niederalkanoylhalogenid, wie -chlorid, in Niederalkanoylamino $R_3$ überführen. Die Umwandlung von Aminogruppen in Amidino-, Guanidino-, Isoharnstoff-, Isothioharnstoff-, Imidoäther- und Imidothioäthergruppen kann beispielsweise nach einem der in der Deutschen Offenlegungsschrift Nr. 26 52 679 genannten Verfahren durchgeführt werden. So können beispielsweise Verbindungen der Formel I, worin $R_3$ Amino darstellt oder $R_4$ durch Amino substituiertes Methyl oder Aethyl ist, durch Umsetzen mit einem Imidohalogenid oder -ester der Formel $[(X_1,Y_1)C{=}X_2'H]^{\oplus}Y_2^{\ominus}$, worin $X_1$ Wasserstoff, Niederalkyl, substituiertes Niederalkyl, Niederalkenyl, Phenyl oder monocyclisches Heteroaryl bedeutet, $X_2'$ gegebenenfalls substituiertes Imino ist, $Y_1$ Halogen, z.B. Chlor, oder Niederalkoxy, z.B. Aethoxy, ist, und $Y_2$ ein Anion, z.B. Chlorid, bedeutet, in Amidine oder durch Umsetzen mit einem Thioharnstoff der Formel $X_1{-}C({=}S){-}X_2$, worin $X_1$ und $X_2$ gleiche oder verschiedene, gegebenenfalls substituierte Aminoreste bedeuten, oder mit einem Isoharnstoff bzw. Isothioharnstoff der Formel $(X_1Y_3)C{=}X_2'$, worin $Y_3$ Niederalkoxy oder Niederalkylthio ist, $X_1$ gegebenenfalls substituiertes Amino und $X_2'$ gegebenenfalls substituiertes Imino bedeutet, in Guanidine übergeführt werden. Weiterhin kann man eine freie Aminogruppe $R_3$ oder Amino als Substituent des Restes $R_4$ in eine durch Niederalkyl mono- oder disubstituierte Aminogruppe überführen. Die Einführung der Niederalkylgruppe(n) erfolgt beispielsweise durch Umsetzung mit entsprechenden reaktionsfähigen Niederalkylestern, wie -halogeniden, z.B. -chloriden oder -bromiden, oder -sulfonaten, z.B. -methan- oder -p-toluolsulfonaten, in Gegenwart eines basischen Kondensationsmittels, wie eines Alkali- oder Erdalkalimetallhydroxids oder -carbonats, z.B. Kaliumhydroxid oder Natriumcarbonat, in einem inerten Lösungsmittel, wie einem Niederalkanol, bei Raumtemperatur oder erhöhter oder erniedrigter Temperatur, z.B. bei etwa -20° bis +80°C.

Salze von Verbindungen der Formel I mit salzbildenden Gruppen können in an sich bekannter Weise hergestellt werden. So kann man Salze von Verbindungen der Formel I mit einer freien Carboxyl- oder Sulfogruppe z.B. durch Behandeln mit Metallverbindungen, wie Alkalimetallsalzen von geeigneten organischen Carbonsäure, z.B. dem Natriumsalz der α-Aethylcapronsäure, oder mit anorganischen Alkali- oder Erdalkalimetallsalzen, z.B. Natriumhydrogencarbonat, oder mit Ammoniak oder mit einem geeigneten organischen Amin bilden, wobei man vorzugsweise stöchiometrische Mengen oder nur einen kleinen Ueberschuss des salzbildenden Mittels verwendet. Säureadditionssalze von Verbindungen der Formel I erhält man in üblicher Weise, z.B. durch Behandeln mit einer geeigneten Säure oder einem geeigneten Anionenaustauscherreagenz. Innere Salze von Verbindungen der Formel I können z.B. durch Neutralisieren von Salzen, wie Säureadditionssalzen, auf den isoelektrischen Punkt, z.B. mit schwachen Basen, oder durch Behandeln mit Ionenaustauschern gebildet werden.

Salze können in üblicher Weise in die freien Verbindungen überführt werden, Metall- und Ammoniumsalze z.B. durch Behandeln mit geeigneten Säuren und Säureadditionssalze z.B. durch Behandeln mit einem geeigneten basischen Mittel.

Erhaltene Gemische von isomeren Verbindungen können nach an sich bekannten Methoden in die einzelnen Isomere aufgetrennt werden. Beispielsweise kann man ein erhaltenes Racemat mit einem optisch aktiven Hilfsstoff reagieren lassen, das dabei entstandene Gemisch zweier diastereomerer Verbindungen mit Hilfe von geeigneten physikalisch-chemischen Methoden (z.B. fraktioniertes Kristallisieren, Adsorptionschromatographie) trennen und die einzelnen diastereomeren Verbindungen dann in die optisch aktiven Verbindungen spalten.
Zur Trennung in Antipoden besonders geeignete Racemate sind solche, die eine saure Gruppe besitzen, wie z.B. Racemate von Verbindungen der Formel I, worin $R_2$ Carboxy ist. Diese sauren Racemate können mit optisch aktiven Basen, z.B. Estern von optisch aktiven Aminosäuren, oder (-)-Brucin, (+)-Chinidin, (-)-Chinin, (+)-Cinchonin, (+)-Dehydroabietylamin, (+)- und (-)-Ephedrin, (+)- und (-)-1-Phenyläthylamin oder deren N-mono- oder N,N-dialkylierten Derivaten zu Gemischen, bestehend aus zwei diastereomeren Salzen, umgesetzt werden.

In Carboxylgruppen enthaltenden Racematen kann diese Carboxylgruppe auch durch einen optisch aktiven Alkohol, wie (-)-Menthol, (+)-Borneol, (+)- oder (-()-2-Octanol verestert werden, worauf nach erfolgter Isolierung des gewünschten Diastereomeren die Carboxylgruppe freigesetzt wird.

Zur Racemattrennung kann auch eine vorhandene Hydroxygruppe mit optisch aktiven Säuren oder deren reaktionsfähigen, funktionellen Derivaten verestert werden, wobei sich diastereomere Ester bilden. Solche Säuren sind beispielsweise (-)-Abietinsäure, D(+)- und L(-)-Aepfelsäure, N-acylierte optisch aktive Aminosäuren, (+)- und (-)-Camphansäure, (+)- und (-)-Ketopinsäure, L(+)-Ascorbinsäure, (+)-Camphersäure, (+)-Campher-10-sulfonsäure(ß), (+)- oder (-)-α-Bromcampher-π-sulfonsäure, D(-)-Chinasäure, D(-)-Isoascorbinsäure, D(-)- und L(+)-Mandelsäure, (+)-1-Menthoxyessigsäure, D(-)- und L(+)-Weinsäure und deren Di-O-Benzoyl- und Di-O-p-Tolylderivate.

Durch Umsetzung mit optisch aktiven Isocyanaten, wie mit (+)- oder (-)-1-Phenyläthylisocyanat, können Verbindungen der Formel (I), worin $R_1$ Hydroxy ist oder $R_4$ durch Hydroxy substituiertes Methyl oder Aethyl ist und die übrigen funktionellen Gruppen geschützt sind, in ein Gemisch diastereomerer Urethane umgewandelt werden.

Basische Racemate, z.B. Verbindungen der Formel I, worin $R_3$ Amino ist oder der Rest $R_4$ durch Amino substituiert ist, können mit den genannten optischen aktiven Säuren diastereomere Salze bilden.

Die Spaltung der aufgetrennten Diastereomeren in die optisch aktiven Verbindungen der Formel I erfolgt ebenfalls nach üblichen Methoden. Aus den Salzen befreit man die Säuren oder die Basen z.B. durch Behandeln mit stärkeren Säuren bzw. Basen als die ursprünglich

eingesetzten. Aus den Estern und Urethanen erhält man die gewünschten optisch aktiven Verbindungen beispielsweise nach alkalischer Hydrolyse oder nach Reduktion mit einem komplexen Hydrid, wie Lithiumaluminiumhydrid.

Eine weitere Methode zur Auftrennung der Racemate besteht in der Chromatographie an optisch aktiven Adsorptionsschichten, beispielsweise an Rohrzucker.

Nach einer dritten Methode können die Racemate in optisch aktiven Lösungsmitteln gelöst und der schwerer lösliche optische Antipode auskristallisiert werden.

Nach einer vierten Methode löst man die Racemate und kristallisiert einen der optischen Antipoden durch Animpfen mit einer kleinen Menge eines nach den obigen Methoden erhaltenen optisch aktiven Produktes aus.

Die Auftrennung der Racemate in die optischen Antipoden kann auf einer beliebigen Verfahrensstufe, d.h. z.B. auch auf der Stufe der Ausgangsverbindungen der Formeln II-VI oder auf einer beliebigen Stufe der nachstehend beschriebenen Verfahren zur Herstellung der Ausgangsverbindungen der Formel II-VI, durchgeführt werden.

Bei allen nachträglichen Umwandlungen erhaltener Verbindungen der Formel I werden solche Reaktionen bevorzugt, die unter schwach alkalischen oder insbesondere neutralen Bedingungen erfolgen.

Das Verfahren umfasst auch diejenigen Ausführungsformen, wonach als Zwischenprodukte anfallende Verbindungen als Ausgangsstoffe verwendet und die restlichen Verfahrensschritte mit diesen durchgeführt werden, oder das Verfahren auf irgendeiner Stufe abgebrochen wird. Ferner können Ausgangsstoffe in Form von Derivaten verwendet oder in situ, gegebenenfalls unter den Reaktionsbedingungen, hergestellt werden.

Die Ausgangsverbindungen der Formeln II und III können, wie in dem folgenden Reaktionsschema I angegeben, hergestellt werden:

Reaktionsschema I

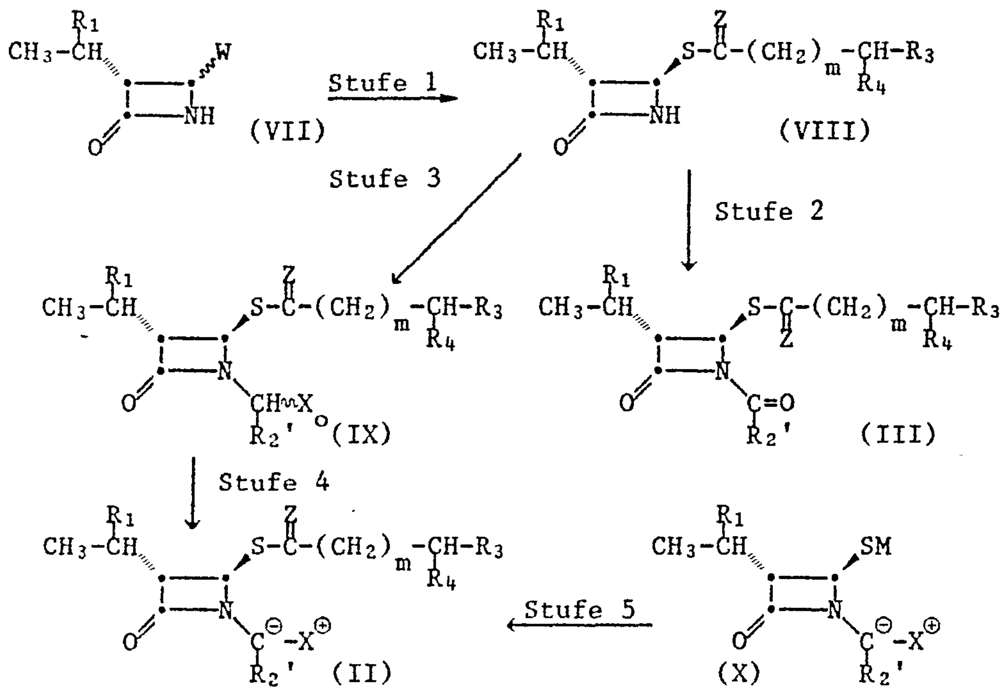


(VII) (VIII) (IX) (III) (II) (X)

Stufe 1

Ein Thioazetidinon der Formel VIII wird erhalten, indem man eine Verbindung der Formel VII mit einer den Rest $-S-C(=Z)-(CH_2)_m-CH(R_3,R_4)$ einführenden Verbindung umsetzt.

In einem Ausgangsmaterial der Formel VII ist W ein nucleofuger, durch die Gruppe $-S-C(=Z)-(CH_2)_m-CH(R_3,R_4)$ ersetzbarer Rest. Solche Reste W sind beispielsweise Acyloxyreste, Sulfonylreste $R_o-SO_2-$, worin $R_o$ ein organischer Rest ist, Azido oder Halogen. In einem Acyloxyrest W ist Acyl z.B. der Rest einer organischen Carbonsäure und bedeutet beispielsweise Niederalkanoyl, z.B. Acetyl oder Propionyl, gegebenenfalls substituiertes Benzoyl, z.B. Benzoyl oder 2,4-Dinitrobenzoyl, oder Phenylniederalkanoyl, z.B. Phenylacetyl. In einem Sulfo-

nylrest $R_o-SO_2-$ ist $R_o$ beispielsweise gegebenenfalls durch Hydroxy substituiertes Niederalkyl, wie Methyl, Aethyl, 2-Hydroxyäthyl, 1-Hydroxyprop-2-yl oder 1-Hydroxy-2-methyl-prop-2-yl, Benzyl oder gegebenenfalls substituiertes Phenyl, wie Phenyl, 4-Bromphenyl oder 4-Methylphenyl. Ein Halogenrest W ist z.B. Brom, Jod oder insbesondere Chlor. W ist bevorzugt Methyl- oder 2-Hydroxyäthylsulfonyl, Acetoxy oder Chlor.

Eine den Rest $-S-C(=Z)-(CH_2)_m-CH(R_3,R_4)$ einführende Verbindung ist beispielsweise eine Säure der Formel $(R_3,R_4)CH-(CH_2)_m-C(=Z)-SH$ oder insbesondere ein Salz, z.B. ein Alkalimetallsalz, wie das Natrium- oder Kaliumsalz, davon. Die Substitution kann in einem organischen Lösungsmittel, wie in einem Niederalkanol, z.B. Methanol oder Aethanol, einem Niederalkanon, z.B. Aceton, einem Niederalkancarbonsäureamid, z.B. Dimethylformamid, einem cyclischen Aether, z.B. Tetrahydrofuran oder Dioxan, oder in einem ähnlichen inerten Lösungsmittel durchgeführt werden. Die Reaktion wird üblicherweise bei Raumtemperatur durchgeführt, kann aber auch bei erhöhter oder erniedrigter Temperatur, z.B. bei etwa 0° bis etwa 40°C, durchgeführt werden. Durch Zugabe eines Salzes der Jodwasserstoffsäure oder der Thiocyansäure, z.B. eines Alkalimetall-, wie Natriumsalzes, kann die Reaktion beschleunigt werden.

Die eintretende Gruppe $-S-C(=Z)-(CH_2)_m-CH(R_3,R_4)$ wird von dem Rest $CH_3-CH(R_1)-$ bevorzugt in die trans-Stellung dirigiert. Daher können sowohl (3S,4R)- als auch (3S,4S)-konfigurierte Ausgangsverbindungen der Formel VII eingesetzt werden. Obwohl die trans-Isomeren überwiegend gebildet werden, können doch gelegentlich auch geringe Mengen des cis-Isomeren entstehen. Die Abtrennung der cis-Isomeren erfolgt, wie oben beschrieben, nach konventionellen Methoden, insbesondere durch Chromatographie und/oder durch Kristallisation.

Geeignete Ausgangsverbindungen der Formel VII sind beispielsweise aus der Europäischen Patentanmeldung Nr. 82113, der Deutschen Offenlegungsschrift Nr. 3 224 055 und der Deutschen Offenlegungsschrift Nr. 3 013 997 bekannt oder können in analoger Weise hergestellt werden. Sie können auch nach den in den Beispielen beschriebenen Verfahren hergestellt worden.

Stufe 2

Eine Ausgangsverbindung der Formel (III) wird erhalten, indem man ein Azetidinon der Formel (VIII) mit einer Säure der Formel $R_2$'-COOH oder insbesondere einem reaktionsfähigen Derivat, wie einem Ester oder Säurehalogenid, z.B. dem Säurechlorid, davon bei einer Temperatur von 20° bis 80°C, bevorzugt bei 40° bis 60°C, in einem inerten Lösungsmittel, wie einem der bei der Umsetzung von Verbindungen der Formel III zu Verbindungen der Formel I genannten, behandelt. Bei Verwendung eines Säurehalogenids arbeitet man vorzugsweise in Gegenwart eines säurebindenden Mittels, wie eines tertiären aliphatischen Amins, z.B. Triäthylamin, eines aromatischen Amins, z.B. Pyridin, oder insbesondere eines Alkalimetall- oder Erdalkalimetallcarbonats oder -hydrogencarbonats, z.B. Kaliumcarbonat oder Calciumcarbonat.

Stufe 3

Verbindungen der Formel IX, worin $X_o$ für eine reaktionsfähige veresterte Hydroxygruppe, insbesondere für Halogen, z.B. Chlor oder Brom, oder organisches Sulfonyloxy, z.B. Niederalkansulfonyloxy, wie Methansulfonyloxy, oder Arensulfonyloxy, z.B. Benzol- oder 4-Methylbenzolsulfonyloxy, steht, werden hergestellt, indem man eine Verbindung der Formel VIII mit einer Glyoxylsäure-Verbindung der Formel OHC-$R_2$' oder einem geeigneten Derivat davon, wie einem Hydrat, Hemihydrat oder Halbacetal, z.B. einem Halbacetal mit einem Niederalkanol, z.B. Methanol oder Aethanol, umsetzt, und in einer erhaltenen Verbindung der Formel IX, worin $X_o$ für Hydroxy steht, die Hydroxygruppe in eine reaktionsfähige veresterte Hydroxygruppe umwandelt.

Die Verbindungen der Formel IX werden üblicherweise als Gemisch der beiden Isomeren [bezüglich der Gruppierung -CH($R_2$')~$X_o$] erhalten.

Man kann aber auch die reinen Isomeren davon isolieren, z.B. durch Chromatographie.

Die Anlagerung der Glyoxylsäureester-Verbindung an das Stickstoffatom des Lactamrings in der Verbindung der Formel VIII findet bei Raumtemperatur oder, falls notwendig, unter Erwärmen, z.B. bei etwa 100°C, und zwar in Abwesenheit eines eigentlichen Kondensationsmittels statt. Bei Verwendung des Hydrats der Glyoxylsäure-Verbindung wird Wasser gebildet, das, wenn notwendig, durch Destillation, z.B. azeotrop, oder durch Verwendung eines geeigneten Dehydratationsmittels, wie eines Molekularsiebs, enfernt wird. Vorzugsweise arbeitet man in Gegenwart eines geeigneten Lösungsmittels, wie z.B. Dioxan, Toluol oder Dimethylformamid, oder Lösungsmittelgemisches, und, wenn erwünscht oder notwendig in der Atmosphäre eines Inertgases, wie Stickstoff.

Die Ueberführung einer Hydroxygruppe $X_o$ in eine reaktionsfähige veresterte Hydroxygruppe $X_o$ in einer Verbindung der Formel IX wird durch Behandeln mit einem geeigneten Veresterungsmittel durchgeführt, z.B. mit einem Thionylhalogenid, z.B. -chlorid, einem Phosphoroxyhalogenid, besonders -chlorid, einem Halogenphosphoniumhalogenid, wie Triphenylphosphoniumdibromid oder -dichlorid oder einem geeigneten organischen Sulfonsäurehalogenid, wie -chlorid, vorzugsweise in Gegenwart eines basischen, in erster Linie organischen basischen Mittels, wie eines aliphatischen tertiären Amins, z.B. Triäthylamin oder Diisopropylamin, oder einer heterocyclischen Base vom Pyridintyp, z.B. Pyridin oder Collidin. Vorzugsweise arbeitet man in Gegenwart eines geeigneten Lösungsmittels, z.B. Dioxan oder Tetrahydrofuran, oder eines Lösungsmittelgemisches, wenn notwendig unter Kühlen, z.B. bei etwa -30° bis etwa 30°C, und gegebenenfalls in der Atmosphäre eines Inertgases, wie Stickstoff.

Stufe 4

Das Ausgangsmaterial der Formel (II) wird erhalten, indem man eine Verbindung der Formel (IX) mit einer geeigneten Phosphin-Verbindung, wie einem Triniederalkylphosphin, z.B. Tri-n-butyl-phosphin, oder einem Triaryl-phosphin, z.B. Triphenylphosphin, oder mit einer geeigneten Phosphit-Verbindung, wie einem Triniederalkylphosphit, z.B. Triäthylphosphit, oder einem Alkalimetalldiniederalkylphosphit, z.B. -diäthylphosphit, behandelt.

Die Umwandlung der Verbindung der Formel (IX) in die Verbindung der Formel (II) wird vorzugsweise in Gegenwart eines geeigneten inerten Lösungsmittels, wie eines Kohlenwasserstoffs, z.B. Cyclohexan oder Benzol, oder eines Aethers, z.B. Dioxan, oder eines Lösungsmittelgemisches vorgenommen. Je nach Reaktionsfähigkeit arbeitet man unter Kühlen oder bei erhöhter Temperatur, etwa zwischen -10° und +100°C, bevorzugt bei etwa 20° bis 80°C, und/oder in der Atmosphäre eines inerten Gases, wie Stickstoff. Zur Verhinderung oxidativer Prozesse können katalytische Mengen eines Antioxidans, z.B. Hydrochinon, zugesetzt werden.

Dabei arbeitet man üblicherweise in Gegenwart eines basischen Mittels, wie einer organischen Base, z.B. eines Amins, wie Triäthylamin, Diisopropyläthylamin, Pyridin, Lutidin, oder "Polystyrol-Hünigbase", oder einer anorganischen Base, z.B. eines Alkalimetallcarbonats, z.B. Natrium- oder Kaliumcarbonat, wobei das primär entstandene Phosphoniumsalz der Formel

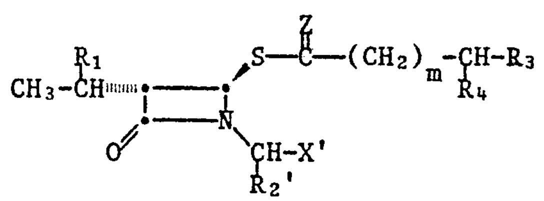

(IIa),

worin X' eine Phosphonogruppe oder eine Phosphoniogruppe zusammen mit einem Anion, je nach Bedeutung des Restes $R_o$ z.B. Chlorid, bedeutet, in das Ylid-Ausgangsmaterial der Formel II umgewandelt

wird. Man kann aber auch in Abwesenheit einer Base arbeiten und eine Verbindung der Formel (IIa), insbesondere eine entsprechende Phosphono-Verbindung, isolieren und diese bei der Herstellung der Endprodukte der Formel (I) in situ in das Ausgangsmaterial der Formel II überführen.

Stufe 5

Eine Verbindung der Formel (II) kann weiterhin erhalten werden, indem man ein Mercaptid der Formel (X), worin M für ein Metallkation steht, mit einem den Rest $(R_3,R_4)CH-(CH_2)_m-C(=Z)$-einführenden Acylierungsmittel behandelt.

In dem Ausgangsmaterial der Formel (X) ist das Metallkation M beispielsweise ein Kation der Formel $M^+$ oder $M^{2+}/2$, wobei $M^+$ insbesondere für ein Silberkation und $M^{2+}$ insbesondere für das zweiwertige Kation eines geeigneten Uebergangmetalls, z.B. Kupfer, Blei oder Quecksilber, steht.

Ein den Rest $(R_3,R_4)CH-(CH_2)_m-C(=Z)-$ einführendes Acylierungsmittel ist z.B. die Säure $(R_3,R_4)CH-(CH_2)_m-C(=Z)-OH$ oder insbesondere ein reaktionsfähiges funktionelles Derivat davon, wie ein Säurehalogenid, z.B. Chlorid oder Bromid, Azid oder Anhydrid davon.

Die Acylierung erfolgt, wenn ein reaktionsfähiges funktionelles Derivat der Säure der Formel $(R_3,R_4)CH-(CH_2)_m-C(=Z)-OH$, z.B. das Säurechlorid, eingesetzt wird, in einem inerten Lösungsmittel, wie einem chlorierten Kohlenwasserstoff, z.B. Methylenchlorid, oder einem Aether, z.B. Diäthyläther oder Dioxan, bei Raumtemperatur oder unter Erhitzen oder Kühlen, z.B. in einem Temperaturbereich von ca. -50° bis ca. +60°C, insbesondere bei ca. -30° bis ca. +20°C.

Die Ausgangsverbindungen der Formel (X) können beispielsweise hergestellt werden, indem man ein Azetidinon der Formel

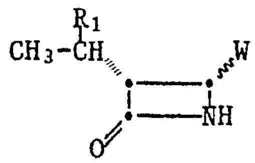

(VII)

durch Umsetzen mit einem Alkalimetallsalz, z.B. dem Natriumsalz, einer Thioniederalkancarbonsäure, z.B. Thioessigsäure, oder des Triphenylmethylmercaptans in eine Verbindung der Formel

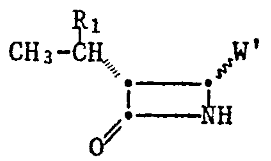

(XI)

überführt, worin W' Triphenylmethylthio oder Niederalkanoylthio, z.B. Acetylthio, bedeutet, diese analog dem in den Reaktionsstufen 3 und 4 beschriebenen Verfahren in eine Verbindung der Formel

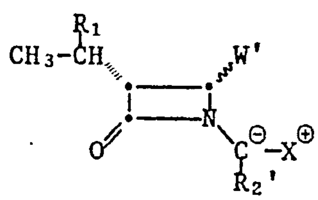

(XII)

überführt und diese in Gegenwart einer Base, z.B. Pyridin oder Tri-n-butylamin, in einem geeigneten Lösungsmittel, z.B. Diäthyläther oder Methanol, mit einem Salz der Formel MA, worin M die obige Bedeutung hat, insbesondere aber für ein Silber-Kation steht, und A ein gebräuchliches Anion darstellt, welches die Löslichkeit des Salzes MA in dem gewählten Lösungsmittel begünstigt, z.B. das Nitrat-, Acetat- oder Fluorid-Anion, umsetzt.

Die Ylide der Formel II können direkt in die Cyclisierungsreaktion zur Herstellung der Endprodukte der Formel I eingesetzt werden. Man kann aber auch in Verbindungen der Formel II, worin $R_1$ eine geschützte Hydroxygruppe, z.B. eine hydrolytisch leicht spaltbare geschützte Hydroxygruppe, wie trisubstituiertes Silyloxy, ist, zuerst die

Hydroxyschutzgruppe abspalten und dann die erhaltene Verbindung der Formel II, worin $R_1$ Hydroxy ist, in die Cyclisierungsreaktion einsetzen.

Die Ausgangsverbindungen der Formel V, worin X für die Gruppe -S-steht, sind bekannt (beispielsweise aus dem Belgischen Patent 898382 und aus Tetrahedron Letters 1983, 1631-1634) oder können in analoger Weise hergestellt werden. Verbindungen der Formel V, worin X für eine Sulfonylgruppe $-SO_2-$ steht, werden hergestellt, indem man eine Verbindung der Formel

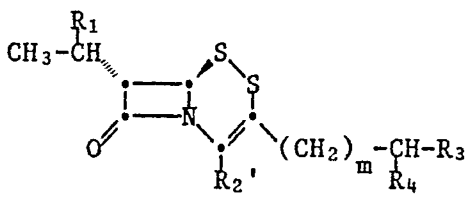

(Va) ,

worin $R_1$, $R_3$, $R_4$ und m die unter Formel I angegebenen Bedeutungen haben und $R_2'$ geschütztes Carboxyl ist, mit einer aliphatischen oder aromatischen Percarbonsäure, z.B. Peressigsäure, m-Chlorperbenzoesäure oder Monoperphthalsäure, in einem inerten Lösungsmittel oder Lösungsmittelgemisch, z.B. in einem Halogenkohlenwasserstoff, z.B. Chloroform, einem Aether oder einem aromatischen Kohlenwasserstoff, z.B. Benzol, bei einer Temperatur von etwa 0° bis etwa 60°C umsetzt.

Die Ausgangsverbindungen der Formel VI können z.B. hergestellt werden, indem man die Verbindung der Formel

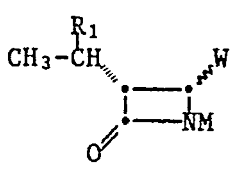

(VII)

mit einer Verbindung der Formel $(R_3',R_4')CH-(CH_2)_m-C(=Z)-SH$ oder einem Salz davon umsetzt und eine erhältliche Verbindung der Formel

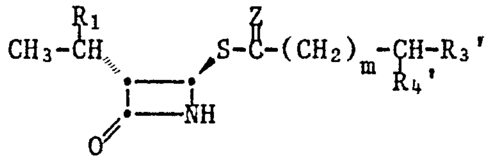

(VIIIa)

nach dem im Reaktionsschema I angegebenen Verfahren in die Ausgangsverbindungen der Formel VI umwandelt.

Man kann die beschriebenen Verfahren zur Herstellung der Verbindungen der Formeln II-VI und VIII-X, sowie die angegebenen Verfahren zur Herstellung der Endprodukte der Formel I auch mit optisch inaktiven Verbindungen durchführen und auf einer beliebigen Verfahrensstufe aus einem erhaltenen Diastereomerengemisch oder Racemat, wie oben beschrieben, die optisch aktiven Verbindungen gemäss der vorliegenden Erfindung isolieren.

Die Erfindung betrifft ebenfalls die neuen Ausgangsverbindungen sowie verfahrensgemäss erhältliche neue Zwischenprodukte, wie diejenigen der Formeln II-VI, VIII und IX sowie die angegebenen Verfahren zu ihrer Herstellung.

Vorzugsweise werden solche Ausgangsverbindungen verwendet und die Reaktionsbedingungen so gewählt, dass man zu den vorstehend als besonders bevorzugt aufgeführten Verbindungen gelangt.

Die pharmakologisch verwendbaren Verbindungen der vorliegenden Erfindung können z.B. zur Herstellung von pharmazeutischen Präparaten verwendet werden, welche eine therapeutisch wirksame Menge der Aktivsubstanz zusammen oder im Gemisch mit anorganischen oder organischen, festen oder flüssigen, pharmazeutisch annehmbaren Trägerstoffen enthalten, die sich zur oralen oder zur parenteralen, d.h. intramuskulären, subcutanen oder intraperitonealen, Verabreichung eignen.

Zur oralen Verabreichung verwendet man Tabletten oder Gelatinekapseln, welche den Wirkstoff zusammen mit Verdünnungsmitteln, z.B. Lactose, Dextrose, Sucrose, Mannitol, Sorbitol, Cellulose und/oder Glycin, und Schmiermitteln, z.B. Kieselerde, Talk, Stearinsäure oder Salze davon, wie Magnesium- oder Calciumstearat, und/oder Polyäthylenglykol, aufweisen. Tabletten enthalten ebenfalls Bindemittel, z.B. Magnesiumaluminiumsilikat, Stärken, wie Mais-, Weizen-, Reis-oder Pfeilwurzstärke, Gelatine, Tragacant, Methylcellulose, Natriumcarboxymethylcellulose und/oder Polyvinylpyrrolidon, und, wenn erwünscht, Sprengmittel, z.B. Stärken, Agar, Alginsäure oder Salze davon, wie Natriumalginat, und/oder Brausemischungen oder Adsorptionsmittel, Farbstoffe, Geschmackstoffe oder Süssmittel.

Zur parenteralen Verabreichung eignen sich in erster Linie Infusionslösungen, vorzugsweise isotonische wässrige Lösungen oder Suspensionen, wobei diese z.B. aus lyophilisierten Präparaten, welche die Wirksubstanz allein oder zusammen mit einem Trägermaterial, z.B. Mannit enthalten, vor Gebrauch hergestellt werden können. Solche Präparate können sterilisiert sein und/oder Hilfsstoffe, z.B. Konservier-, Stabilisier-, Netz- und/oder Emulgiermittel, Löslichkeitsvermittler, Salze zur Regulierung des osmotischen Druckes und/oder Puffer enthalten.

Die vorliegenden pharmazeutischen Präparate, die, wenn erwünscht, weitere pharmakologisch wertvolle Stoffe enthalten können, werden in an sich bekannter Weise, z.B. mittels konventionellen Mischungs-, Lösungs- oder Lyophilisierungsverfahren, hergestellt und enthalten von etwa 0,1 % bis 100 %, insbesondere von etwa 1 % bis etwa 50 %, Lyophilisate bis zu 100 % des Aktivstoffs.

Je nach Art der Infektion und Zustand des infizierten Organismus verwendet man tägliche Dosen (oral oder parenteral) von etwa 100 mg bis etwa 500 mg zur Behandlung von Warmblütern (Menschen und Tiere) von etwa 70 kg Gewicht.

Die genannten pharmazeutischen Präparate und die Verwendung der erfindungsgemässen Verbindungen der Formel I zur therapeutischen Behandlung des menschlichen und tierischen Körpers bilden ebenfalls einen Gegenstand der vorliegenden Erfindung.

Die folgenden Beispiele dienen zur Illustration der Erfindung. Temperaturen werden in Celsiusgraden angegeben.

In den Beispielen werden folgende Abkürzungen verwendet:

DC: Dünnschichtchromatogramm
IR: Infrarotspektrum
UV: Ultraviolettspektrum
THF: Tetrahydrofuran

Beispiel 1: (5R,6S)-2-[(2R,S)-2-Allyloxycarbonylamino-prop-1-yl]-6-[(1R)-1-allyloxycarbonyloxyäthyl]-2-penem-3-carbonsäure-allylester

Eine Lösung von 2 g 2-[(3S,4R)-3-[(1R)-1-Allyloxycarbonyloxyäthyl]-4-[(3R,S)-3-allyloxycarbonylaminobutyroylthio]-2-oxo-azetidin-1-yl]-2-triphenylphosphoranylidenessigsäure-allylester in 400 ml Toluol wird unter Argonatmosphäre 6,5 Stunden bei Rückflusstemperatur gerührt. Dann wird das Lösungsmittel abgedampft und das Rohprodukt durch Chromatographie an Silicagel gereinigt (Laufmittel Toluol/Aethylacetat 4:1), $R_f = 0,54$; IR ($CH_2Cl_2$) 3430; 1790; 1740; 1730; 1580 $cm^{-1}$.

Das Ausgangsmaterial kann wie folgt hergestellt werden:

a) (3R,S)-3-Allyloxycarbonylaminobuttersäure

7,83 g (3R,S)-3-Aminobuttersäure wird in 17 ml Wasser und 35 ml 5N Natriumhydroxid gelöst und nach Abkühlen durch ein Eisbad mit 8,88 ml Chlorameisensäureallylester versetzt. Nach Entfernen des Kühlbades wird das Reaktionsgemisch während 16 Stunden bei Raumtemperatur gerührt, dann anschliessend mit 150 ml Wasser verdünnt und zweimal mit Aethylacetat gewaschen. Die wässrige Phase wird mit 4N Salzsäure-

Lösung auf pH 2 gestellt und zweimal mit Aethylacetat extrahiert. Die organischen Extrakte werden einmal mit Sole gewaschen, über $MgSO_4$ getrocknet und eingedampft. IR ($CH_2Cl_2$) 3440; 1715 $cm^{-1}$.

b) (3R,S)-3-Allyloxycarbonylaminobuttersäurechlorid

1,87 g (3R,S)-3-Allyloxycarbonylaminobuttersäure wird bei 0° mit 2,85 ml Thionylchlorid versetzt. Nach 1,25 Stunden Rühren bei 0° wird das Reaktionsgemisch dreimal mit Toluol eingedampft. Die rohe Titelverbindung [IR ($CH_2Cl_2$): 3440; 1795; 1720 $cm^{-1}$] wird gleich weiter umgesetzt.

c) 2-{(3S,4R)-3-[(1R)-1-Allyloxycarbonyloxyäthyl]-4-[(3R,S)-3-allyloxycarbonylaminobutyroylthio]-2-oxo-azetidin-1-yl}-2-triphenylphosphoranylidenessigsäureallylester

2,79 g Silbersalz des 2-{(3S,4R)-3-[(1R)-1-Allyloxycarbonyloxyäthyl]-4-mercapto-2-oxo-azetidin-1-yl}-2-triphenylphosphoranylidenessigsäureallylesters werden in 25 ml Methylenchlorid gelöst, bei 0° mit 0,97 ml Pyridin und anschliessend tropfenweise mit einer Lösung von 1,23 g (3R,S)-3-Allyloxycarbonylaminobuttersäurechlorid in 10 ml Methylenchlorid versetzt. Nach 30 Minuten Rühren bei 0° wird der Feststoff über Hyflo abfiltriert und das Filtrat wird mit wässriger Natriumbicarbonat-Lösung und dann mit Sole gewaschen. Nach Trocknen über $MgSO_4$ wird das Lösungsmittel eingedampft. Der Rückstand wird durch Chromatographie an Silicagel (Laufmittel Toluol-Aethylacetat 3:2) gereinigt. DC (Silicagel, Toluol/Aethylacetat 2:3), $R_f$ = 0,38; IR ($CH_2Cl_2$) 3440; 1755; 1725; 1625 $cm^{-1}$.

Das Silbersalz des 2-{(3S,4R)-3-[(1R)-1-Allyloxycarbonyloxyäthyl]-4-mercapto-2-oxoazetidin-1-yl}-2-triphenylphosphoranylidenessigsäure-allylesters wird folgendermassen hergestellt:

ca) (2S,3R)-2-Brom-3-hydroxybuttersäure-p-methoxybenzylamid

Zu einer bei Raumtemperatur unter Argonatmosphäre gerührten Lösung von 5 g (2S,3R)-2-Brom-3-hydroxybuttersäure und 3,52 g p-Methoxybenzylamin in 60 ml abs. THF werden innerhalb 20 Minuten 4,16 g 1-Hydroxybenztriazol und 5,63 g Dicyclohexylcarbodiimid in 60 ml THF

zugetropft. Man rührt das Reaktionsgemisch 48 Stunden, filtriert den ausgefallenen Dicyclohexylharnstoff ab, wäscht diesen mehrmals mit THF und dampft das Filtrat ein. Das erhaltene Rohprodukt enthält Dicyclohexylharnstoff und Hydroxybenztriazol als Verunreinigung. Nach Chromatographieren des Gemisches an Silicagel (System: Toluol; Toluol/Aethylacetat 1:4) und Kristallisation der reinen Fraktionen aus Methylenchlorid/Diäthyläther wird die Titelverbindung vom F. 122-124° erhalten. [α] = -7 ±1° (1,112 % in Chloroform).

cb) (2R,3R)-2,3-Epoxybuttersäure-p-methoxybenzylamid

Eine Lösung von 6,04 g (20 mM) (2S,3R)-2-Brom-3-hydroxybuttersäure-p-methoxybenzylamid in 150 ml Methylenchlorid wird mit 50 ml 50%iger NaOH-Lösung un 456 mg (2 mM) Benzyltriäthylammoniumchlorid versetzt. Das zweiphasige Gemisch wird während 20 Stunden bei Raumtemperatur stark gerührt. Die organische Schicht wird abgetrennt und die wässrige Phase mit Methylenchlorid nachextrahiert. Die vereinigten Methylenchlorid-Lösungen werden getrocknet und eingedampft. Das anfallende Rohprodukt wird an 40-facher Gewichtsmenge Silicagel im System Methylenchlorid/Methanol (99:1) chromatographiert. Nach Kristallisation der reinen Fraktionen aus Methylenchlorid-Diäthyläther-Petrolether wird die Titelverbindung, F.: 75-76°, erhalten.

cc) (2R,3R)-2,3-Epoxybuttersäure-N-tert-butoxycarbonylmethyl-N-p-methoxybenzylamid

Zu einer bei 0° unter Argonatmospähre gerührten Mischung von 550 mg Natriumhydrid-Dispersion (55-60 % in Oel) und 1,52 ml Bromessigsäure-tert-butylester in 25 ml THF wird eine Lösung von 2,21 g (2R,3R)-2,3-Epoxybuttersäure-p-methoxybenzylamid in 100 ml THF zugetropft. Man erwärmt auf Raumtemperatur und rührt das Reaktionsgemisch 1 Stunde weiter (Kontrolle des Reaktionsablaufs mittels Dünnschichtchromatographie). Gesamtreaktionszeit: 90 Minuten. Die unlöslichen Anteile werden abfiltriert und mit THF gewaschen und die vereinigten Filtrate eingedampft. Das anfallende Rohprodukt reinigt man durch Chromatographieren an 150 g Silicagel (System: Toluol,

Toluol/Aethylacetat 80:20). Durch Eindampfen der reinen Fraktionen erhält man die amorphe Titelverbindung. IR-Spektrum: Banden u.a. bei 1740, 1670, 1650, 1615, 1517, 1465, 1360 und 1035 $cm^{-1}$.

cd) (3S,4S)-1-(p-Methoxybenzyl)-3-[(1R)-1-hydroxyäthyl]-4-tert-butoxycarbonyl-2-azetidinon

9,23 g Tetrabutylammoniumfluorid-trihydrat werden mit 40 g Molekularsieb (Typ 4171/16 - bei 300° vorgetrocknet) in 80 ml THF 16 Stunden bei 5° stehen gelassen. Die Mischung wird auf 0° abgekühlt, mit einer Lösung von 2,8 g (2R,3R)-2,3-Epoxybuttersäure-N-tert-butoxycarbonylmethyl-N-p-methoxybenzylamid in 20 ml THF versetzt und 2 Stunden bei 0-5° gerührt. Das Molekularsieb wird unter Nachwaschen mit THF abfiltriert und das Filtrat direkt auf eine in Toluol bereitete Säule mit 250 g Silicagel aufgetragen. Die eingedampften, mit Toluol/Aethylacetat (70:30-Gemisch) eluierten Fraktionen werden in Methylenchlorid aufgenommen, nacheinander zweimal mit 1N Schwefelsäure, mit gesättigter, wässriger $NaHCO_3$-Lösung und mit Wasser gewaschen, getrocknet und eingedampft. Nach Kurzchromatographie an Silicagel (Toluol, Toluol/Aethylacetat 60:40) und Kristallisation aus Methylenchloid/Diäthyläther/Petroläther wird die reine Titelverbindung vomF. 85-87° erhalten.

ce) (3S,4S)-1-(p-Methoxybenzyl)-3-[(1R)-1-allyloxycarbonyloxyäthyl]-4-tert-butoxycarbonyl-2-azetidinon

Eine Lösung von 2 g (3S,4S)-1-(p-Methoxybenzyl)-3-[(1R)-1-hydroxyäthyl)-4-tert-butoxycarbonyl-2-azetidinon in 24 ml Methylenchlorid wird bei 0° mit 24 ml 1N NaOH, 820 mg Tetrabutylammoniumhydrogensulfat und 1 ml Chlorameisensäureallylester versetzt und heftig gerührt. Nach 20 und 40 Minuten Reaktionszeit werden weitere Portionen (je 1 ml) Chlorameisensäureallylester zugegeben. Das Reaktionsgemisch wird mit Methylenchlorid verdünnt, die wässrige Phase abgetrennt und die organische Schicht nacheinander mit 5%-iger wässriger Zitronensäure und 8%-iger wässriger $NaHCO_3$-Lösung gewaschen, getrocknet und eingedampft. Nach chromatographischer Reinigung wird die reine, amorphe Titelverbindung. IR-Spektrum: Banden u.a. bei 1765, 1745(sh), 1615, 1593, 1515, 1160 und 1035 $cm^{-1}$.

cf) (3S,4S)-1-(p-Methoxybenzyl)-3-[(1R)-1-allyloxycarbonyloxyäthyl]-4-carbonsäure-2-azetidinon

1,6 g (3S,4S)-1-(p-Methoxybenzyl)-3-[(1R)-1-allyoxycarbonyloxyäthyl]-4-tert-butoxycarbonyl-2-azetidinon werden bei 0° in 10 ml Trifluoressigsäure gelöst. Nach einer Stunde Reaktionszeit bei Raum temperatur wird das Reaktionsgemisch im Hochvakuum eingedampft und die erhältliche Titelverbindung ohne Reinigung weiterverarbeitet. [α]= +85 ±1° (1,0 % in Chloroform).

cg) (3R,4R)-1-(p-Methoxybenzyl)-3-[(1R)-1-allyloxycarbonyloxyäthyl]-4-acetoxy-2-azetidinon

Eine unter Argonatmosphäre bei Raumtemperatur gerührte Lösung von 1,4 g (3S,4S)-1-(p-Methoxybenzyl)-3-[(1R)-1-allyloxycarbonyloxyäthyl]-4-carbonsäure-2-azetidinon in einem Gemisch aus 45 ml THF und 6,6 ml Dimethylformamid wird mit 1,6 g Blei-(IV)-acetat (ca. 10 % Essigsäure-Gehalt) versetzt und ca. 1 Stunde bis zur vollständigen Umsetzung des Substrats gerührt. Ueberschüssiges Oxidationsmittel wird durch Zugabe von 0,5 ml Aethylenglykol zersetzt (10 Min Raumtemperatur). Das Reaktionsgemisch wird von ausgefallenem Blei-(II)-acetat abfiltriert, der Filtrierrückstand mit THF ausgewaschen und das Filtrat eingedampft. Der erhaltene ölige Rückstand wird in Methylenchlorid aufgenommen, nacheinander je zweimal mit gesättigter, wässriger $NaHCO_3$-Lösung, Wasser und ges. NaCl-Lösung gewaschen, getrocknet und eingedampft. Durch Chromatographieren des Rückstands an Silicagel (Toluol; Toluol/Aethylacetat 90:10) wird die reine Titelverbindung [α]= +90° ±1° (1,0 % in Chloroform) erhalten.

ch) (3R,4R)-3-[(1R)-1-Allyloxycarbonyloxyäthyl]-4-acetoxy-2-azetidinon

Eine Lösung von 900 mg (1,18 mMol) (3R,4R)-1-(p-Methoxybenzyl)-3-[1R)-1-allyloxycarbonyloxyäthyl]-4-acetoxy-2-azetidinon in 30 ml Acetonitril wird bei 10° mit einer Lösung von 5,37 g Cer-(IV)-ammoniumnitrat in 15 ml Wasser versetzt und zwei Stunden bei Raumtemperatur gerührt. Nach Extraktion mit Aethylacetat, Waschen mit gesättigter $NaHCO_3$-Lösung, Trocknen der organischen Phase über Natriumsulfat und Eindampfen unter vermindertem Druck erhält man die rohe Titelverbindung, welche man chromatographisch an Silicagel mit Toluol-Aethylacetat (4:1 und 1:1) reinigt. [α]= +84 ±1° (1,0 % in Chloroform).

ci) (3S,4R)-3-[(1R)-1-Allyloxycarbonyloxyäthyl]-4-triphenylmethylthio-azetidin-2-on

892 mg Triphenylmethylmercaptan werden in 5 ml Methanol bei 0° suspendiert und über 10 Minuten portionsweise mit insgesamt 0,16 g einer 50%igen Natriumhydrid-Suspension in Oel versetzt. Anschliessend wird eine Lösung von 0,62 g (3S,4R)-3-[(1R)-1-Allyloxycarbonyloxy-äthyl]-4-acetoxy-azetidin-2-on in 7 ml Aceton und 5 ml Wasser über 15 Minuten zugetropft. Nach 1 Stunde Rühren bei 0° und drei weiteren Stunden bei Raumtemperatur wird das Reaktionsgemisch am Rotationsverdampfer eingeengt und mit 20 ml Methylenchlorid extrahiert. Die organische Phase wird mit Sole gewaschen und über $MgSO_4$ getrocknet. Nach Einengen wird die rohe Titelverbindung durch Chromatographie auf Silicagel (Laufmittel Toluol-Aethylacetat 19:1) gereineigt.
DC (Toluol-Aethylacetat 4:1) $R_f$ = 0,3, IR ($CH_2Cl_2$) 3400; 1770 1745 $cm^{-1}$.

cj) 2-{(3S,4R)-3-[(1R)-1-Allyloxycarbonyloxyäthyl]-4-triphenylmethylthio-2-oxo-azetidin-1-yl}-2-hydroxyessigsäure-allylester

0,82 g (3S,4R)-3-[(1R)-1-Allyloxycarbonyloxyäthyl]-4-triphenylmethylthio-azetidin-2-on und 0,416 g Glyoxylsäureallylester-äthylhemiacetal in 10 ml abs. Toluol werden mit 4 g Molekularsieb (4Å) versetzt und während 61 Stunden bei 60° gerührt. Nach Abfiltrieren und Einengen am Rotationsverdampfer unter vermindertem Druck wird die Titelverbindung erhalten. DC (Silicagel/Toluol/Aethylacetat 4:1), $R_f$ = 0,1. IR ($CH_2Cl_2$): 3510; 1770; 1745; $cm^{-1}$.

ck) 2-{(3S,4R)-3-[(1R)-1-Allyloxycarbonyloxyäthyl]-4-triphenylmethylthio-2-oxo-azetidin-1-yl}-2-triphenylphosphoranyliden-essigsäure-allylester

Zu einer Lösung von 1 g 2-{(3S,4R)-3-[(1R)-1-Allyloxycarbonyloxy-äthyl]-4-triphenylmethylthio-2-oxo-azetidin-1-yl}-2-hydroxy-

essigsäure-allylester in 10 ml Tetrahydrofuran werden unter Rühren bei 0° nacheinander 0,182 ml Thionylchlorid und 0,206 ml Pyridin innert 5 Minuten zugegeben. Die weisse Suspension wird 30 Minuten bei 0° gerührt und über Hyflo filtriert. Nach Waschen des Rückstands mit Toluol wird am Rotationsverdampfer eingeengt. Der Rückstand wird in 10 ml Dioxan gelöst, mit 0,624 g Triphenylphosphin und 0,257 ml Lutidin versetzt und während 46 Stunden bei 80° Badtemperatur gerührt. Das Gemisch wird über Hyflo filtriert und der Rückstand mit Toluol nachgewaschen. Die vereinten Filtrate werden eingedampft und die Chromatographie des Rückstandes an Silicagel ergibt das reine Produkt (Laufmittel Toluol/Aethylacetat 19:1 bis 4:1), DC (Silicagel; Toluol/Aethylacetat 4:1), $R_f$ = 0,24, IR ($CH_2Cl_2$) 1745; 1620 $cm^{-1}$.

c1) Silbersalz des 2-{(3S,4R)-3-[(1R)-1-Allyloxycarbonyloxyäthyl]-4-triphenylmethylthio-2-oxo-azetidin-1-yl}-2-triphenylphosphoranyliden-essigsäureallylesters

0,46 g 2-{(3S,4R)-3-[(1R)-1-Allyloxycarbonyloxyäthyl]-4-triphenylmethylthio-2-oxo-azetidin-1-yl}-2-triphenylphosphoranylidenessigsäureallylester werden in 6 ml Diäthyläther vorgelegt und bei Raumtemperatur mit 4,4 ml einer 0,5M wässrigen Silbernitrat-Lösung versetzt. Danach gibt man 0,077 ml Triäthylamin dazu und rührt das Reaktionsgemisch während 30 Minuten nach. Der Feststoff wird abgenutscht und gut mit Wasser und Diäthyläther gewaschen. Der Rückstand wird nochmals in 300 ml Wasser und 300 ml Diäthyläther aufgeschlämmt, gerührt und dann abfiltriert. Nach erneutem Waschen mit Diäthyläther wird der Feststoff am Hochvakuum getrocknet. IR ($CH_2Cl_2$) 1760; 1745; 1630 $cm^{-1}$.

Beispiel 2: (5R,6S)-2-[(2R,S)-2-Amino-prop-1-yl]-6-[(1R)-1-hydroxyäthyl]-2-penem-3-carbonsäure

1,05 g (5R,6S)-2-[(2R,S)-2-Allyloxycarbonylamino-prop-1-yl]-6-[(1R)-1-allyloxycarbonyloxyäthyl]-2-penem-3-carbonsäureallylester werden in 28 ml abs. Tetrahydrofuran vorgelegt und nacheinander mit 105 mg Tetrakis-(triphenylphosphin)-palladium und portionsweise mit 2,03 ml Tri-n-butylzinnhydrid versetzt. Nach 2 Stunden Rühren bei Raumtemperatur wird das Reaktionsgemisch mit 0,813 ml Essigsäure

versetzt. Nach weiteren 30 Minuten Rühren bei Raumtemperatur werden 40 ml Hexan dazugegeben. Der Feststoff wird abfiltriert und anschliessend in Wasser gelöst. Die Lösung wird mit wenig Aktivkohle geklärt und über Minipor-Filter filtriert. Die Titelverbindung wird durch Lyophilisation erhalten. UV (Phosphatpuffer pH 7,4) $\lambda_{max}$ = 304,5 nm, IR (DMSO·$d_6$): 1772 $cm^{-1}$.

Das entstandene Diastereomeren-Gemisch, bestehend aus Verbindungen mit einer (2R)-2-Aminoprop-1-yl- bzw. einer (2S)-2-Aminoprop-1-yl-Gruppe in 2-Stellung, kann durch Säulenchromatographie (Antecgel OPTI UP $C_{12}$; Laufmittel:Wasser) in seine Komponenten aufgetrennt werden:

Isomeres A: $R_f$ (Antecgel OPTI-UP$C_{12}$, Wasser) = 0,2.
NMR (360 MHz; $D_2O$; Referenz TSP) : $\delta$ = 1,3 (3H,d), 1,37 (3H,d) 2,95 und 3,18 (2H,m), 3,6 (1H,m), 3,9 (1H,dd), 4,25 (1H,m) und 5,7 ppm (1H,d).

Isomeres B: $R_f$ (Antecgel OPTI-UP$C_{12}$, Wasser) = 0,17
NMR (360 MHz; $D_2O$; Referenz TSP): $\delta$ = 1,3 (3H,d), 1,39 (3H,d), 2,97 und 3,27 (2H,m), 3,65 (1H,m), 3,92 (1H,dd), 4,26 (1H,m) und 5,67 ppm (1H,d).

Beispiel 3: (5R,6S)-2-[(2R,S)-2-Allyloxycarbonylamino-but-1-yl]-6-[(1R)-1-allyloxycarbonyloxyäthyl]-2-penem-3-carbonsäure-allylester

Analog Beispiel 1 werden 1,98 g 2-{(3S,4R)-3-[(1R)-1-Allyloxycarbonyloxyäthyl]-4-[(3R,S)-3-allyloxycarbonylaminovaleroylthio]-2-oxo-azetidin-1-yl}-2-triphenylphosphoranylidenessigsäure-allylester in die Titelverbindung übergeführt. IR ($CH_2Cl_2$): 3440; 1790; 1740; 1720; 1710; 1580 $cm^{-1}$.

Das Ausgangsmaterial kann wie folgt hergestellt werden:

a) (3R,S)-3-Allyloxycarbonylaminovaleriansäure

Analog Beispiel 1a) werden 5,86 g (3R,S)-3-Aminovaleriansäure in die Titelverbindung überführt. IR ($CH_2Cl_2$): 3430; 1710 $cm^{-1}$.

b) (3R,S)-3-Allyloxycarbonylaminovaleriansäurechlorid

Analog Beispiel 1b) werden 0,4 g (3R,S)-3-Allyloxycarbonylaminovaleriansäure in die Titelverbindung überführt. IR ($CH_2Cl_2$): 3430; 1785; 1710 $cm^{-1}$.

c) 2-{(3S,4R)-3-[(1R)-1-Allyloxycarbonyloxyäthyl]-4-[(3R,S)-3-allyloxycarbonylaminovaleroylthio]-2-oxo-azetidin-1-yl}-2-triphenylphosphoranyliden-essigsäureallylester

Analog Beispiel 1c) werden 2,79 g Silbersalz des 2-{(3S,4R)-3-[(1R)-1-Allyloxycarbonyloxyäthyl]-4-mercapto-2-oxoazetidin-1-yl}-2-triphenylphosphoranylidenessigsäureallylesters mit 1,32 g (3R,S)-3-Allyloxycarbonylaminovaleriansäurechlorid in die Titelverbindung überführt. IR ($CH_2Cl_2$) 3440; 1760; 1725; 1625 $cm^{-1}$.

Beispiel 4: (5R,6S)-2-[(2R,S)-2-Amino-but-1-yl)-6-[(1R)-1-hydroxyäthyl]-2-penem-3-carbonsäure

Analog Beispiel 2 werden 0,8 g (5R,6S)-2-[(2R,S)-2-Allyloxycarbonylamino-but-1-yl]-6-[(1R)-1-allyloxycarbonyloxyäthyl]-2-penem-3-carbonsäure-allylester in die Titelverbindung überführt.
UV (Phosphatpuffer pH 7,4): $\lambda_{max}$ = 306 nm.

Beispiel 5: (5R,6S)-2-[(2R,S)-2-N-Formamidinoprop-1-yl]-6-[(1R)-1-hydroxyäthyl]-2-penem-3-carbonsäure

Eine Lösung von 109,5 mg Aethyl-formimidat-hydrochlorid und 84 mg Natriumhydrogencarbonat in 4 ml Wasser werden bei Raumtemperatur mit einer Lösung von 25 mg (5R,6S)-2-[(2R,S)-2-Amino-prop-1-yl]-6-[(1R)-1-hydroxyäthyl]-2-penem-3-carbonsäure und 8,4 mg Natriumbicarbonat in 1 ml Wasser versetzt. Nach 50 min Rühren bei Raumtemperatur wird mit 1 ml 1N HCl versetzt und am Hochvakuum eingedampft. Die Rohsubstanz wird durch Chromatographie am OPTI-$UPC_{12}$ gereinigt.
UV (Phosphatpuffer pH 7,4): $\lambda_{max}$ 304,5 nm.

Beispiel 6: (5R,6S)-2-[(2R,S)-2-Aminoprop-1-yl]-6-[(1R)-1-hydroxyäthyl]-2-penem-3-carbonsäure-1-äthoxycarbonyl-oxyäthylester

1,2 g Natriumjodid werden in 3,7 ml Aceton gelöst und mit 0,275 ml Aethyl-1-chloräthylcarbonat versetzt. Das Gemisch wird bei Raumtemperatur während 3 Stunden gerührt. Anschliessend wird die Lösung auf 10,5 ml Methylenchlorid getropft und von den ausgefallenen anorganischen Salzen abfiltriert. Die Methylenchloridlösung wird bis auf 2 ml eingeengt und bei 0° zu einer Lösung von 0,272 g (1 mMol) (5R,6S)-2-[2R,S)-2-Aminoprop-1-yl]-6-[(1R)-1-hydroxyäthyl]-2-penem-3-carbonsäure in 4 ml Dimethylacetamid gegeben. Dann wird während 3 Stunden bei 0° gerührt, anschliessend mit Aethylacetat verdünnt und dreimal mit Wasser gewaschen. Die organischen Phasen werden über Natriumsulfat getrocknet und am Rotationsverdampfer eingeengt. Das Rohprodukt wird an 10 g Silicagel mit dem Laufmittel Aethylacetat gereinigt. Man erhält die Titelverbindung als weissen Schaum. IR-Spektrum (Methylenchlorid): Absorptionsbanden bei 1790 und 1740 $cm^{-1}$.

Beispiel 7: (5R,6S)-2-[(2R,S)-2-Aminoprop-1-yl]-6-[(1R)-1-hydroxyäthyl]-2-penem-3-carbonsäure-pivaloyl-oxymethylester

0,6 g Natriumjodid werden in 2 ml Aceton gelöst und mit 0,15 ml Pivalinsäurechlormethylester versetzt. Das Gemisch wird bei Raumtemperatur während 3 Stunden gerührt und anschliessend auf 7,5 ml Methylenchlorid getropft. Die ausgefallenen anorganischen Salze werden abfiltriert. Die Methylenchloridlösung wird bis auf 1 ml eingeengt und zu einer Lösung von 0,11 g (0,4 mMol) (5R,6S)-2-[(2R,S)-2-Aminoprop-1-yl]-6-[(1R)-1-hydroxyäthyl]-2-penem-3-carbonsäure und 0,07 ml Diisopropyläthylamin in 4 ml N,N-Dimethylacetamid bei 0° gegeben. Dann wird während 3 Stunden bei 0° gerührt, anschliessend mit Aethylacetat verdünnt und dreimal mit Wasser gewaschen. Die organische Phase wird über Natriumsulfat getrocknet und am Rotationsverdampfer eingeengt. Das Rohprodukt wird an 10 g

Silicagel mit dem Laufmittel Aethylacetat gereinigt. Man erhält die Titelverbindung als weissen Schaum. IR-Spektrum (Methylenchlorid): Absorptionsbadnen bei 1790 und 1730 $cm^{-1}$.

Beispiel 8: In analoger Weise, wie in den vorstehenden Beispielen beschrieben, können die folgenden Verbindungen hergestellt werden:

(5R,6S)-2-[(3R,S)-3-Aminobut-1-yl]-6-[(1R)-1-hydroxyäthyl]-2-penem-3-carbonsäure
UV (Phosphatpuffer pH 7,4) $\lambda_{max}$ = 305 nm,

(5R,6S)-2-[(2R,S)-2-Amino-3-hydroxy-prop-1-yl]-6-[(1R)-1-hydroxyäthyl]-2-penem-3-carbonsäure
UV (Phosphatpuffer pH 7,4) $\lambda_{max}$ = 304 nm,

(5R,6S)-2-[(2R,S)-2,3-Diamino-prop-1-yl]-6-[(1R)-1-hydroxyäthyl]-2-penem-3-carbonsäure
UV (Phosphatpuffer pH 7,4) $\lambda_{max}$ = 305,5 nm,

(5R,6S)-2-[(2R,S)-2-N-Methylaminoprop-1-yl]-6-[(1R)-1-hydroxyäthyl]-2-penem-3-carbonsäure
UV (Phosphatpuffer pH 7,4) $\lambda_{max}$ = 306 nm,

(5R,6S)-2-[(2R,S)-2-N-Formylamino-prop-1-yl]-6-[(1R)-1-hydroxyäthyl]-2-penem-3-carbonsäure
UV (Phosphatpuffer pH 7,4) $\lambda_{max}$ = 305 nm,

(5R,6S)-2-[(2R,S)-2-N-Acetylamino-prop-1-yl]-6-[(1R)-1-hydroxyäthyl]-2-penem-3-carbonsäure
UV (Phosphatpuffer pH 7,4) $\lambda_{max}$ = 304,5 nm,

Beispiel 9: Trockenampullen oder Vials, enthaltend (5R,6S)-2-[(2R,S)-2-Amino-prop-1-yl]-6-[(1R)-1-hydroxyäthyl]-2-penem-3-carbonsäure als Wirksubstanz, werden wie folgt hergestellt:

Zusammensetzung (für 1 Ampulle oder Vial):

| | |
|---|---|
| Wirksubstanz | 0,5 g |
| Mannit | 0,05 g |

Eine sterile wässrige Lösung der Wirksubstanz und des Mannits wird unter aseptischen Bedingungen in 5 ml-Ampullen oder 5 ml-Vials der Gefriertrocknung unterworfen und die Ampullen bzw. Vials verschlossen und geprüft.

Anstelle des obengenannten Wirkstoffs kann auch dieselbe Menge eines anderen Wirkstoffs der vorangehenden Beispiele, wie z.B. (5R,6S)-2-[(2R,S)-2-Amino-but-1-yl]-6-[(1R)-1-hydroxyäthyl]-2-penem-3-carbonsäure, verwendet werden.

Patentansprüche für die Vertragsstaaten BE, FR, DE, CH, LI, IT, LU, NL, SE, GB

1. Verbindungen der Formel

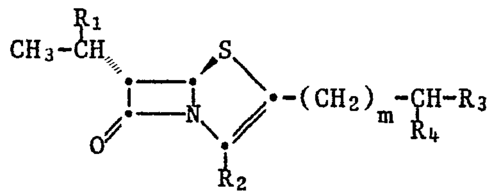

(I) ,

worin $R_1$ Hydroxy oder geschütztes Hydroxy ist, $R_2$ Carboxyl oder funktionell abgewandeltes Carboxyl bedeutet, $R_3$ Amino, durch Niederalkyl substituiertes Amino, Niederalkanoylamino, substituiertes Methylenamino oder geschütztes Amino ist, $R_4$ gegebenenfalls durch Amino, durch Niederalkyl substituiertes Amino, substituiertes Methylenamino, geschütztes Amino, Hydroxy oder geschütztes Hydroxy substituiertes Methyl oder Aethyl ist, und m eine ganze Zahl von 1 bis 3 ist, optische Isomere von Verbindungen der Formel I, Mischungen solcher optischen Isomere und Salze von solchen Verbindungen der Formel I, die eine salzbildende Gruppe aufweisen.

2. Verbindungen der Formel I gemäss Anspruch 1, worin $R_1$ Hydroxy oder geschütztes Hydroxy ist; $R_2$ Carboxyl, unter physiologischen Bedingungen spaltbares verestertes Carboxyl oder geschütztes Carboxyl $R_2'$ ist, $R_3$ Amino, Niederalkylamino, Diniederalkylamino, Niederalkanoylamino, eine Gruppe der Formel $-N{=}C(X_1,X_2)$, worin $X_1$ Wasserstoff, Amino, Niederalkylamino, Diniederalkylamino, Niederalkylenamino, Nitroamino, Hydrazino, Anilino, Niederalkoxy, Phenylniederalkoxy, Niederalkylthio, Niederalkyl, Aminoniederalkyl, N-Niederalkylaminoniederalkyl, N,N-Diniederalkylaminoniederalkyl, Niederalkenyl, Phenyl oder über ein Ringkohlenstoffatom gebundenes monocyclisches 5- oder 6-gliedriges Heteroaryl mit 1 oder 2 Stickstoffatomen und/oder einem Sauerstoff- oder Schwefelatom, ist, und $X_2$ Amino, Niederalkylamino, Diniederalkylamino, Niederalkylenamino,

Hydrazino, Anilino, Niederalkoxy, Phenylniederalkoxy oder Niederalkylthio ist, oder geschütztes Amino darstellt; $R_4$ Methyl, Aethyl oder durch Amino, Niederalkylamino, Diniederalkylamino, eine Gruppe der Formel $-N=C(X_1,X_2)$, worin $X_1$ und $X_2$ die bei der Definition von $R_3$ angegebenen Bedeutungen haben, geschütztes Amino, Hydroxy oder geschütztes Hydroxy substituiertes Methyl oder Aethyl bedeutet, und m eine ganze Zahl von 1 bis 3 ist, optische Isomere von Verbindungen der Formel I, Mischungen dieser optischen Isomere und Salze von solchen Verbindungen der Formel I, die eine salzbildende Gruppe aufweisen.

3. Verbindungen der Formel I gemäss Anspruch 1, worin $R_1$ Hydroxy ist, $R_2$ Carboxyl oder unter physiologischen Bedingungen spaltbares verestertes Carboxyl, z.B. Niederalkanoyloxymethoxycarbonyl oder 1-Niederalkoxycarbonyloxyniederalkoxycarbonyl, bedeutet, $R_3$ Amino, Niederalkylamino, Diniederalkylamino, Niederalkanoylamino oder eine Gruppe der Formel $-N=C(X_1,X_2)$, worin $X_1$ Wasserstoff, Niederalkyl oder Amino und $X_2$ Amino ist, darstellt, $R_4$ Methyl, Aethyl oder durch Amino, Niederalkylamino oder Hydroxy substituiertes Methyl oder Aethyl ist, und m 1 oder 2 bedeutet, optische Isomere von Verbindungen der Formel I, Mischungen dieser optischen Isomere und Salze von solchen Verbindungen der Formel I, die eine salzbildende Gruppe aufweisen.

4. Verbindungen der Formel I gemäss Anspruch 1, worin $R_1$ Hydroxy ist, $R_2$ Carboxyl oder unter physiologischen Bedingungen spaltbares verestertes Carboxyl, z.B. Niederalkanoyloxymethoxycarbonyl oder 1-Niederalkoxycarbonyloxyniederalkoxycarbonyl, darstellt, $R_3$ Amino ist, $R_4$ Methyl oder Aethyl bedeutet und m 1 oder 2 ist, optische Isomere von Verbindungen der Formel I, Mischungen dieser optischen Isomere und Salze von solchen Verbindungen der Formel I, die eine salzbildende Gruppe aufweisen.

5. Die pharmazeutisch annehmbaren Salze von Verbindungen der Formel I gemäss Anspruch 1.

6. Die unter physiologischen Bedingungen spaltbaren Ester von Verbindungen der Formel I gemäss Anspruch 1.

7. Verbindungen der Formel I gemäss Anspruch 1, worin der Rest $-CH(R_1)(CH_3)$ die R-Konfiguration besitzt.

8. (5R,6S)-2-[(2R,S)-2-Amino-prop-1-yl]-6-[(1R)-1-hydroxyäthyl]-2-penem-3-carbonsäure, pharmazeutisch annehmbare Salze davon und unter physiologischen Bedingungen spaltbare Ester davon gemäss Anspruch 1.

9. (5R,6S)-2-[(2R)-2-Aminoprop-1-yl]-6-[(1R)-1-hydroxyäthyl]-2-penem-3-carbonsäure, pharmazeutisch annehmbare Salze davon und unter physiologischen Bedingungen spaltbare Ester davon gemäss Anspruch 1.

10. (5R,6S)-2-[(2S)-2-Amino-prop-1-yl]-6-[(1R)-1-hydroxyäthyl]-2-penem-3-carbonsäure, pharmazeutisch annehmbare Salze davon und unter physiologischen Bedingungen spaltbare Ester davon gemäss Anspruch 1.

11. (5R,6S)-2-[(2R,S)-2-Amino-but-1-yl)-6-[(1R)-1-hydroxy-äthyl]-2-penem-3-carbonsäure, die optischen Isomere davon, pharmazeutisch annehmbare Salze davon und unter physiologischen Bedingungen spaltbare Ester davon gemäss Anspruch 1.

12. Pharmazeutisches Präparat enthaltend eine Verbindung der Formel I gemäss Anspruch 1, ein pharmazeutisch annehmbares Salz oder einen unter physiologischen Bedingungen spaltbaren Ester einer solchen Verbindung.

13. Verwendung von Verbindungen der Formel I gemäss Anspruch 1, von pharmazeutisch annehmbaren Salzen und von unter physiologischen Bedingungen spaltbaren Estern solcher Verbindungen zur Herstellung von pharmazeutischen Präparaten.

14. Eine Verbindung der Formel I gemäss Anspruch 1, pharmazeutisch annehmbare Salze und unter physiologischen Bedingungen spaltbare Ester von solchen Verbindungen als antibiotische Mittel.

15. Eine Verbindung der Formel I gemäss Anspruch 1, pharmazeutisch annehmbare Salze und unter physiologischen Bedingungen spaltbare Ester von solchen Verbindungen zur Anwendung in einem Verfahren zur therapeutischen Behandlung des menschlichen oder tierischen Körpers.

16. Verfahren zur Herstellung von Verbindungen der Formel I gemäss Anspruch 1, dadurch gekennzeichnet, dass man

a) eine Ylid-Verbindung der Formel

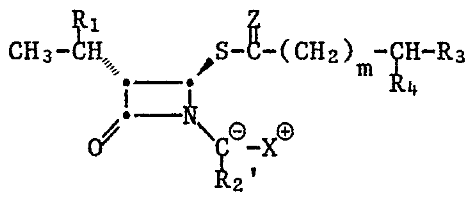

(II) ,

worin $R_1$, $R_3$, $R_4$ und m die unter Formel I angegebenen Bedeutungen haben, $R_2'$ geschütztes Carboxyl ist, Z Sauerstoff oder Schwefel bedeutet und $X^{\oplus}$ entweder eine dreifach substituierte Phosphoniogruppe oder eine zweifach veresterte Phosphonogruppe zusammen mit einem Kation darstellt, ringschliesst, oder

b) eine Verbindung der Formel

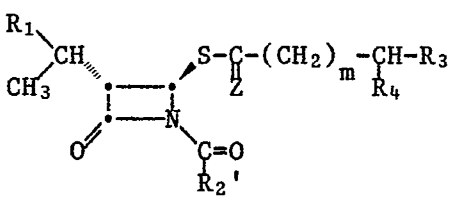

(III) ,

worin $R_1$, $R_3$, $R_4$ und m die unter Formel I angegebenen Bedeutungen haben, $R_2'$ geschütztes Carboxyl ist und Z Sauerstoff oder Schwefel ist, mit einer organischen Verbindung des dreiwertigen Phosphors behandelt, oder

c) in einer Verbindung der Formel

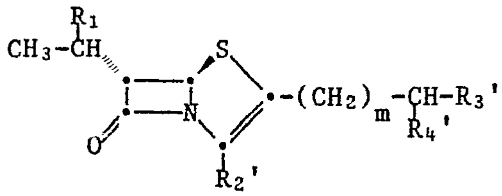

(IV) ,

worin $R_1$ und m die unter Formel I angegebenen Bedeutungen haben, $R_2'$ eine geschützte Carboxylgruppe ist, und $R_3'$ eine durch den Rest $R_3$ ersetzbare Gruppe oder $R_3$ und $R_4'$ eine in den Rest $R_4$ überführbare Gruppe oder $R_4$ ist, mit der Massgabe, dass mindestens eine der Gruppen $R_3'$ und $R_4'$ von $R_3$ bzw. $R_4$ verschieden ist, die Gruppe $R_3'$ durch den Rest $R_3$ ersetzt und/oder die Gruppe $R_4'$ in den Rest $R_4$ überführt oder

d) in einer Verbindung der Formel

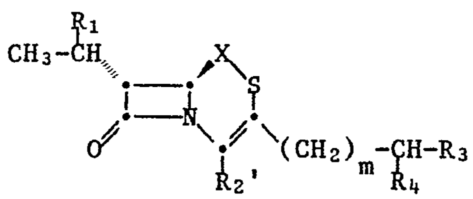

(V) ,

worin $R_1$, $R_3$, $R_4$ und m die unter Formel I angegebenen Bedeutungen haben, $R_2'$ eine geschützte Carboxylgruppe darstellt und X für die Gruppe -S- oder für die Gruppe $-SO_2-$ steht, den Rest X abspaltet, und, wenn erwünscht oder notwendig, in einer erhältlichen Verbindung der Formel I eine geschützte Hydroxygruppe $R_1$ oder geschütztes Hydroxy im Rest $R_4$ in die freie Hydroxygruppe überführt, und/oder, wenn erwünscht, in einer erhältlichen Verbindung der Formel I eine geschützte Carboxylgruppe $R_2'$ in die freie Carboxylgruppe, in eine unter physiologischen Bedingungen spaltbare veresterte Carboxylgruppe oder in eine andere geschützte Carboxylgruppe $R_2'$ oder eine freie Carboxylgruppe $R_2$ in eine unter physiologischen Bedingungen spaltbare veresterte Carboxylgruppe überführt, und/oder, wenn erwünscht, eine geschützte Aminogruppe $R_3$ oder geschütztes Amino im Rest $R_4$ in die freie Aminogruppe oder eine freie Aminogruppe $R_3$ oder freies Amino im Rest $R_4$ in eine substituierte Aminogruppe überführt, und/oder, wenn erwünscht, ein erhältliches Gemisch von

isomeren Verbindungen der Formel I in die einzelnen Isomere auftrennt, und/oder, wenn erwünscht, eine erhältliche Verbindung mit salzbildender Gruppe in ein Salz oder ein erhältliches Salz in die freie Verbindung oder in ein anderes Salz überführt.

17. Die nach dem Verfahren gemäss Anspruch 16 erhältlichen Verbindungen.

Patentansprüche für den Vertragsstaat AT

1. Verfahren zur Herstellung von Verbindungen der Formel

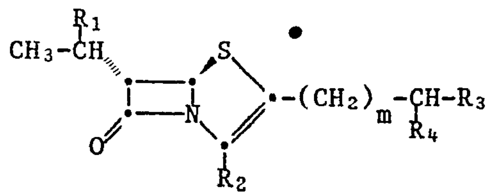

(I) ,

worin $R_1$ Hydroxy oder geschütztes Hydroxy ist, $R_2$ Carboxyl oder funktionell abgewandeltes Carboxyl bedeutet, $R_3$ Amino, durch Niederalkyl substituiertes Amino, Niederalkanoylamino, substituiertes Methylenamino oder geschütztes Amino ist, $R_4$ gegebenenfalls durch Amino, durch Niederalkyl substituiertes Amino, substituiertes Methylenamino, geschütztes Amino, Hydroxy oder geschütztes Hydroxy substituiertes Methyl oder Aethyl ist, und m eine ganze Zahl von 1 bis 3 ist, optische Isomere von Verbindungen der Formel I, Mischungen solcher optischen Isomere und Salze von solchen Verbindungen der Formel I, die eine salzbildende Gruppe aufweisen, dadurch gekennzeichnet, dass man

a) eine Ylid-Verbindung der Formel

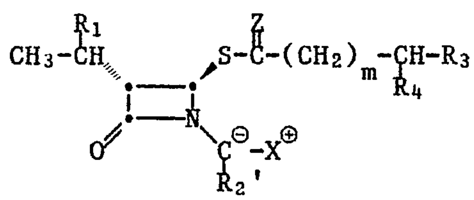

(II) ,

worin $R_1$, $R_3$, $R_4$ und m die unter Formel I angegebenen Bedeutungen haben, $R_2'$ geschütztes Carboxyl ist, Z Sauerstoff oder Schwefel bedeutet und $X^\oplus$ entweder eine dreifach substituierte Phosphoniogruppe oder eine zweifach veresterte Phosphonogruppe zusammen mit einem Kation darstellt, ringschliesst, oder

b) eine Verbindung der Formel

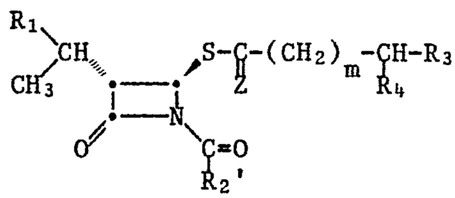

(III) ,

worin $R_1$, $R_3$, $R_4$ und m die unter Formel I angegebenen Bedeutungen haben, $R_2'$ geschütztes Carboxyl ist und Z Sauerstoff oder Schwefel ist, mit einer organischen Verbindung des dreiwertigen Phosphors behandelt, oder

c) in einer Verbindung der Formel

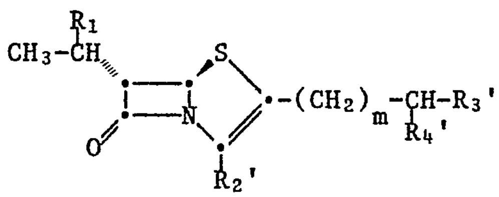

(IV) ,

worin $R_1$ und m die unter Formel I angegebenen Bedeutungen haben, $R_2'$ eine geschützte Carboxylgruppe ist, und $R_3'$ eine durch den Rest $R_3$ ersetzbare Gruppe oder $R_3$ und $R_4'$ eine in den Rest $R_4$ überführbare Gruppe oder $R_4$ ist, mit der Massgabe, dass mindestens eine der Gruppen $R_3'$ und $R_4'$ von $R_3$ bzw. $R_4$ verschieden ist, die Gruppe $R_3'$ durch den Rest $R_3$ ersetzt und/oder die Gruppe $R_4'$ in den Rest $R_4$ überführt, oder

d) in einer Verbindung der Formel

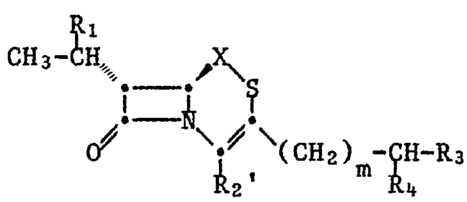

(V) ,

worin $R_1$, $R_3$, $R_4$ und m die unter Formel I angegebenen Bedeutungen haben, $R_2'$ eine geschützte Carboxylgruppe darstellt und X für die Gruppe -S- oder für die Gruppe $-SO_2-$ steht, den Rest X abspaltet, und, wenn erwünscht oder notwendig, in einer erhältlichen Verbindung der Formel I eine geschützte Hydroxygruppe $R_1$ oder geschütztes

Hydroxy im Rest $R_4$ in die freie Hydroxygruppe überführt, und/oder, wenn erwünscht, in einer erhältlichen Verbindung der Formel I eine geschützte Carboxylgruppe $R_2'$ in die freie Carboxylgruppe, in eine unter physiologischen Bedingungen spaltbare veresterte Carboxylgruppe oder in eine andere geschützte Carboxylgruppe $R_2'$ oder eine freie Carboxylgruppe $R_2$ in eine unter physiologischen Bedingungen spaltbare veresterte Carboxylgruppe überführt, und/oder, wenn erwünscht, eine geschützte Aminogruppe $R_3$ oder geschütztes Amino im Rest $R_4$ in die freie Aminogruppe oder eine freie Aminogruppe $R_3$ oder freies Amino im Rest $R_4$ in eine substituierte Aminogruppe überführt, und/oder, wenn erwünscht, ein erhältliches Gemisch von isomeren Verbindungen der Formel I in die einzelnen Isomere auftrennt, und/oder, wenn erwünscht, eine erhältliche Verbindung mit salzbildender Gruppe in ein Salz oder ein erhältliches Salz in die freie Verbindung oder in ein anderes Salz überführt.

2. Verfahren nach Anspruch 1 zur Herstellung von Verbindungen der Formel I, worin $R_1$ Hydroxy oder geschütztes Hydroxy ist; $R_2$ Carboxyl, unter physiologischen Bedingungen spaltbares verestertes Carboxyl oder geschütztes Carboxyl $R_2'$ ist, $R_3$ Amino, Niederalkylamino, Diniederalkylamino, Niederalkanoylamino, eine Gruppe der Formel $-N=C(X_1,X_2)$, worin $X_1$ Wasserstoff, Amino, Niederalkylamino, Diniederalkylamino, Niederalkylenamino, Nitroamino, Hydrazino, Anilino, Niederalkoxy, Phenylniederalkoxy, Niederalkylthio, Niederalkyl, Aminoniederalkyl, N-Niederalkylaminoniederalkyl, N,N-Diniederalkylaminoniederalkyl, Niederalkenyl, Phenyl oder über ein Ringkohlenstoffatom gebundenes monocyclisches 5- oder 6-gliedriges Heteroaryl mit 1 oder 2 Stickstoffatomen und/oder einem Sauerstoff- oder Schwefelatom, ist, und $X_2$ Amino, Niederalkylamino, Diniederalkylamino, Niederalkylenamino, Hydrazino, Anilino, Niederalkoxy, Phenylniederalkoxy oder Niederalkylthio ist, oder geschütztes Amino darstellt; $R_4$ Methyl, Aethyl oder durch Amino, Niederalkylamino, Diniederalkylamino, eine Gruppe der Formel $-N=C(X_1,X_2)$, worin $X_1$ und $X_2$ die bei der Definition von $R_3$ angegebenen Bedeutungen haben, geschütztes Amino, Hydroxy oder geschütztes Hydroxy substituiertes Methyl oder Aethyl bedeutet, und m eine ganze Zahl von 1 bis 3 ist,

optische Isomere von Verbindungen der Formel I, Mischungen dieser optischen Isomere und Salze von solchen Verbindungen der Formel I, die eine salzbildende Gruppe aufweisen.

3. Verfahren nach Anspruch 1 zur Herstellung von Verbindungen der Formel I, worin $R_1$ Hydroxy ist, $R_2$ Carboxyl oder unter physiologischen Bedingungen spaltbares verestertes Carboxyl, z.B. Niederalkanoyloxymethoxycarbonyl oder 1-Niederalkoxycarbonyloxyniederalkoxycarbonyl, bedeutet, $R_3$ Amino, Niederalkylamino, Diniederalkylamino, Niederalkanoylamino oder eine Gruppe der Formel $-N=C(X_1,X_2)$, worin $X_1$ Wasserstoff, Niederalkyl oder Amino und $X_2$ Amino ist, darstellt, $R_4$ Methyl, Aethyl oder durch Amino, Niederalkylamino oder Hydroxy substituiertes Methyl oder Aethyl ist, und m 1 oder 2 bedeutet, optische Isomere von Verbindungen der Formel I, Mischungen dieser optischen Isomere und Salze von solchen Verbindungen der Formel I, die eine salzbildende Gruppe aufweisen.

4. Verfahren nach Anspruch 1 zur Herstellung von Verbindungen der Formel I, worin $R_1$ Hydroxy ist, $R_2$ Carboxyl oder unter physiologischen Bedingungen spaltbares verestertes Carboxyl, z.B. Niederalkanoyloxymethoxycarbonyl oder 1-Niederalkoxycarbonyloxyniederalkoxycarbonyl, darstellt, $R_3$ Amino ist, $R_4$ Methyl oder Aethyl bedeutet und m 1 oder 2 ist, optische Isomere von Verbindungen der Formel I, Mischungen dieser optischen Isomere und Salze von solchen Verbindungen der Formel I, die eine salzbildende Gruppe aufweisen.

5. Verfahren nach Anspruch 1 zur Herstellung von pharmazeutisch annehmbaren Salze von Verbindungen der Formel I.

6. Verfahren nach Anspruch 1 zur Herstellung von unter physiologischen Bedingungen spaltbaren Ester von Verbindungen der Formel I.

7. Verfahren nach Anspruch 1 zur Herstellung von Verbindungen der Formel I, worin der Rest $-CH(R_1)(CH_3)$ die R-Konfiguration besitzt.

8. Verfahren nach Anspruch 1 zur Herstellung von (5R,6S)-2-[(2R,S)-2-Amino-prop-1-yl]-6-[(1R)-1-hydroxyäthyl]-2-penem-3-carbonsäure, pharmazeutisch annehmbaren Salzen davon und unter physiologischen Bedingungen spaltbaren Estern davon.

9. Verfahren nach Anspruch 1 zur Herstellung von (5R,6S)-2-[(2R)-2-Aminoprop-1-yl]-6-[(1R)-1-hydroxyäthyl]-2-penem-3-carbonsäure, pharmazeutisch annehmbaren Salzen davon und unter physiologischen Bedingungen spaltbaren Estern davon.

10. Verfahren nach Anspruch 1 zur Herstellung von (5R,6S)-2-[(2S)-2-Amino-prop-1-yl]-6-[(1R)-1-hydroxyäthyl]-2-penem-3-carbonsäure, pharmazeutisch annehmbaren Salzen davon und unter physiologischen Bedingungen spaltbaren Estern davon.

11. Verfahren nach Anspruch 1 zur Herstellung von (5R,6S)-2-[(2R,S)-2-Amino-but-1-yl)-6-[(1R)-1-hydroxy-äthyl]-2-penem-3-carbonsäure, otpischen Isomeren davon, pharmazeutisch annehmbaren Salzen davon und unter physiologischen Bedingungen spaltbaren Estern davon.

12. Verfahren zur Herstellung von pharmazeutischen Präparaten, dadurch gekennzeichnet, dass man eine gemäss Anspruch 1 erhältliche Verbindung der Formel I mit einem pharmazeutisch annehmbaren Trägerstoff mischt.

FO 7.4/UL/we*

0171362

Europäisches Patentamt

**EUROPÄISCHER TEILRECHERCHENBERICHT,**
der nach Regel 45 des Europäischen Patentübereinkommens für das weitere Verfahren als europäischer Recherchenbericht gilt

Nummer der Anmeldung
EP 85 81 0360

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | EP-A- 0 070 204 (SUMITOMO)<br>* Seiten 57,58, Beispiel 34; Ansprüche 1-13 *<br>--- | 1-6,12, 16 | C 07 D 499/00<br>A 61 K 31/43//<br>C 07 F 9/65<br>C 07 D 205/08 |
| P,X | EP-A- 0 125 208 (CIBA-GEIGY)<br>* Ansprüche *<br>-------- | 1-14, 16,17 | |

RECHERCHIERTE SACHGEBIETE (Int. Cl.4)
C 07 D 499/00
A 61 K 31/00

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 1-14,16,17
Unvollständig recherchierte Patentansprüche:
Nicht recherchierte Patentansprüche: 15
Grund für die Beschränkung der Recherche: Verfahren zur chirurgischen oder therapeutischen Behandlung des menschlichen oder tierischen Körpers (Siehe Art. 52(4) des Europäisches Patentübereinkommens).

| Recherchenort | Abschlußdatum der Recherche | Prüfer |
|---|---|---|
| Den Haag | 18-09-1985 | CHOULY |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument

EPA Form 1505.1 03 82